# EUROPEAN PATENT APPLICATION

(11) **EP 3 378 494 A1**
(43) Date of publication of application: **26.09.2018**
(21) Application number: 16866237.7
(22) Date of filing: 10.11.2016
(51) Int. Cl.: A61K 47/50, A61K 31/706, A61P 29/00, A61P 37/02

(54) **POLYMERIC DERIVATIVE OF MACROLIDE IMMUNOSUPPRESSANT**

(30) Priority: 18.11.2015 JP 2015225900
(71) Applicant: Nippon Kayaku Kabushiki Kaisha, Tokyo 100-0005 (JP)
(72) Inventor: SEKIGUCHI Akihiro, Tokyo 115-8588 (JP); MIZUNUMA Kana, Tokyo 115-8588 (JP); YAMAMOTO Keiichirou, Tokyo 115-8588 (JP); YONEKI Nao, Tokyo 115-8588 (JP); HAYASHI Tomohiro, Tokyo 115-8588 (JP); SAIGA Kan, Tokyo 115-8588 (JP); KONNO Junpei, Tokyo 115-8588 (JP); KOBAYASHI Yuki, Tokyo 115-8588 (JP); SATO Takamichi, Tokyo 115-8588 (JP); IGO Naoko, Tokyo 115-8588 (JP); FUCHIGAMI Kimiko, Tokyo 115-8588 (JP)
(74) Representative: Ter Meer Steinmeister & Partner
(86) International application number: PCT/JP2016/083417
(87) International publication number: WO 2017/086235

(57) **Abstract**

A problem of the present invention is to obtain a preparation which shows markedly enhanced effectiveness and safety by exhibiting high accumulability at diseased areas and having the blood concentration maintained at a constant level without individual differences, and for which the control of the dosage depending on the blood kinetics (blood trough concentration) becomes unnecessary. The invention relates to a polymeric derivative of tacrolimus including a copolymer of a polyethylene glycol segment and a polymeric moiety, in which a carboxy group in a side chain of the copolymer is bonded to an alcoholic hydroxy group of tacrolimus.

## Description

### Technical Field

The present invention relates to polymeric derivatives of macrolide compounds, methods for preparing the derivatives, and use of the derivatives.

### Background Art

The macrolide compounds used for the present invention share common activity of having an affinity to FKBP type immunophilin and of inhibiting the enzymatic activity of peptidyl-prolyl isomerases and/or rotamases. Examples of the macrolide compounds include tricyclo compounds including rapamycin, tacrolimus (FK506), ascomycin, and the like.

It is described in, for example, Patent Literature 1, that macrolide compounds or pharmaceutically acceptable salts thereof have excellent immunosuppressive action, antibacterial activity, and other pharmacological activity, and therefore, those compounds are useful for the treatment and prevention of rejection to a transplant of an organ or a tissue, a graft-versus-host reaction, an autoimmune disease, an infectious disease, and the like.

Tacrolimus is generally used for the suppression of a rejection after organ transplantation and for the treatment of a graft-versus-host disease after bone marrow transplantation, intractable active phase ulcerative colitis, and the like. However, tacrolimus is sparingly soluble in water (2.4 to 3.6 µM, room temperature) and has low bioavailability at the time of oral administration. Furthermore, since the therapeutic range of tacrolimus is narrow, and the inter-individual and intra-individual variation in the pharmacokinetics is large, tacrolimus is a drug for which control of the blood concentration is difficult. Some known causative factors for the variation in the pharmacokinetics of tacrolimus include low solubility in water, individual differences in the expression level and the genotype of P-glycoprotein or drug metabolizing enzyme, CYP3A, in the alimentary canal mucosa, and the like (Non-Patent Literatures 1 and 2).

One of main side effects of tacrolimus is renal toxicity and pancreatic toxicity. Renal toxicity is caused by the occurrence of decreases in the blood flow rate and the glomerular filtration rate resulting from the vasoconstrictor action on renal arterioles, and stagnation of nutritional supplementation to the tubular cells. Pancreatic toxicity is the onset of impaired glucose tolerance caused by suppression of the insulin production from pancreatic β cells mainly based on the inhibition of insulin mRNA transcription in pancreatic β cells. These toxicities are expressed dependently on the concentration of tacrolimus in blood plasma.

Regarding side effects of tacrolimus on the central nerve system, reversible posterior encephalopathy syndrome, hypertensive encephalopathy, and the like have been reported to occur in human subjects (0.1% to less than 0.5%, including post-marketing surveillance). Furthermore, it has been recognized in rats that intravenous administration of tacrolimus causes slight respiratory distress, a decrease in locomotor activity, a prone position, stereotyped behavior, and the like (Non-Patent Literature 3).

It is known that when a polymer and a drug are coupled, the drug has increased water-solubility or improved pharmacokinetics in vivo, and as a result, excellent effects in practical use, such as enhanced efficacy, reduced side effects, and sustainability of efficacy, are obtained. The coupling modes for a polymer and a drug include a case in which a polymer and a drug are covalently bonded to each other, and a case in which a polymer and a drug are physically adsorbed to each other. A drug covalently bonded to a polymer is released from the polymer in vivo by means of a hydrolysis reaction or the like. Meanwhile, a drug physically adsorbed to a polymer does not depend on any chemical reaction such as a hydrolysis reaction, and the drug is slowly released from the polymer in vivo. In both cases, no enzyme is involved in the release of the drug from the polymer; however, it is considered that the difference in the coupling mode for the polymer and the drug brings about differences in the mechanism of drug release.

Patent Literature 2 and Patent Literature 3 describe polymeric derivatives obtainable from a drug and a copolymer including a polyethylene glycol and polyaspartic acid. In the polymeric derivatives described in Patent Literature 2 and Patent Literature 3, the copolymer and the drug are physically adsorbed to each other. Sustained release of the polymeric derivative is attributed to slow dissociation of the drug from the copolymer, and thus, drug efficacy is selectively exhibited at diseased areas, while side effects occur at a reduced level.

Patent Literature 4 discloses a method of obtaining a compound in which tacrolimus is physically adsorbed to a polymer including an alkyl-substituted polylactide (MPEG-hexPLA). However, there is no description with regard to the blood concentration or tacrolimus migrating specifically to diseased areas.

In Non-Patent Literature 4, a PEGylated tacrolimus obtained by chemically bonding tacrolimus to a PEG polymer has been reported. However, the PEGylated tacrolimus did not give an effect surpassing the effect of tacrolimus in inflammatory disease model animals such as an adjuvant arthritis mouse and a lupus nephritis mouse.

In Non-Patent Literature 5, micelles synthesized by physically adsorbing tacrolimus to a poly(ethylene glycol) esters-polycaprolactone (PEG-PCL) polymer have been reported. It has been found that the PEG-PCL tacrolimus micelles have high inflammation ameliorating effects such as suppression of bodyweight reduction, suppression of shortening of the large intestine, hemorrhage of the large intestine, and loss of crypt cells, with respect to a DSS-induced ulcerative colitis mouse, compared to tacrolimus. However, since the micelles should be administered continuously once a day for 12 days in order for the PEG-PCL tacrolimus micelles to exhibit an inflammation ameliorating effect, it is considered that the blood concentration may not be maintained for a long time with this compound.

In Non-Patent Literatures 6 and 7, micelles synthesized by physically adsorbing tacrolimus to a polymer including polycaprolactone-b-poly(ethylene oxide) (PCL-b-PEO) have been reported. It is described that the PCL-b-PEO tacrolimus micelles are slowly taken into cells compared to tacrolimus. Furthermore, it is disclosed that when the PCL-b-PEO tacrolimus micelles were intravenously administered through the caudal vein at a dose of 5 mg/kg three times at an interval of 6 days, spontaneous motility of a sciatic nerve injury model rat was improved. However, since tacrolimus released from the PCL-b-PEO tacrolimus micelles highly accumulates in the brain, it is considered that the PCL-b-PEO tacrolimus micelles are specialized in the function as a neuroprotective agent, rather than the function as an immunosuppressant.

In Non-Patent Literature 8, nanoparticles synthesized by physically adsorbing tacrolimus to a polymer including poly(lactic-co-glycolic acid) (PLGA) or pH-sensitive Eudragit P-4135F have been reported. These nanoparticles exhibit higher inflammation ameliorating effects than tacrolimus in collagen-induced arthritis and DSS-induced colitis mice. However, since the nanoparticles should be continuously administered once a day for 12 days in order for the nanoparticles to exhibit an inflammation ameliorating effect, it is considered that the blood concentration may not be maintained for a long time with this compound. Furthermore, the ameliorating action against BUN, serum creatinine, and creatinine clearance, which are indicators for nephropathy, are also not sufficient.

To this date, a preparation which shows markedly enhanced effectiveness and safety compared to tacrolimus, by exhibiting high accumulability at diseased areas and having the blood concentration maintained at a constant level without individual differences, and for which the control of the dosage depending on the blood kinetics (blood trough concentration) becomes unnecessary, has not yet been developed, and there is a demand for the development of such a preparation.

### Citation List

### Patent Literature

Patent Literature 1: WO 93/005059 A
Patent Literature 2: WO 2003/000771 A
Patent Literature 3: WO 2004/082718 A
Patent Literature 4: WO 2013/157664 A

### Non Patent Literature

Non-Patent Literature 1: Transplantation, 1999, 67, p. 333-335
Non-Patent Literature 2: Pharm. Res., 1998, 15, p. 1609-1613
Non-Patent Literature 3: Materials on PROGRAPH capsules (0.5 mg, 1 mg, 5 mg) and PROGRAPH injectable solution (5 mg) of Fujisawa Pharmaceutical Co., Ltd. (currently Astellas Pharma Inc.), p. 53, Summary of application materials for department meeting deliberation and approval of June 2001
Non-Patent Literature 4: Arch. Pharm. Res., 2011, 34, 1301-1310
Non-Patent Literature 5: J. Biomed. Nanotechnol., 2013, 9, p. 147-157
Non-Patent Literature 6: Drug. Deliv., 2000, 7, p. 139-145
Non-Patent Literature 7: Biochim. Biophys. Acta, 1999, 1421, p. 32-38
Non-Patent Literature 8: Int. J. Pharm., 2006, 316, p. 138-143

### Summary of Invention

### Technical Problem

An object of the present invention is to provide a novel immunosuppressant or an anti-inflammatory agent, which accumulates the drug at an inflammation site, exhibits a superior effect with a low dosage, has a long administration interval and reduces toxicity by maintaining the target blood trough concentration.

### Solution to Problem

The inventors of the present invention found that a polymeric derivative of tacrolimus including a copolymer of a polyethylene glycol segment and a polyamino acid derivative, in which a side-chain carboxy group of the copolymer is bonded to an alcoholic hydroxy group of tacrolimus, solves the problems of the present invention.

The present invention relates to the following items [1] to [18].
[1] A polymeric derivative of tacrolimus, including a copolymer of a polyethylene glycol segment and a polyamino acid derivative, wherein a carboxy group in a side chain of the copolymer is bonded to an alcoholic hydroxy group of tacrolimus.
[2] The polymeric derivative of tacrolimus according to [1], wherein the polyamino acid derivative is a polyaspartic acid derivative.
[3] The polymeric derivative of tacrolimus according to [1] or [2], wherein the polymeric derivative of tacrolimus is represented by the following General Formula (1): 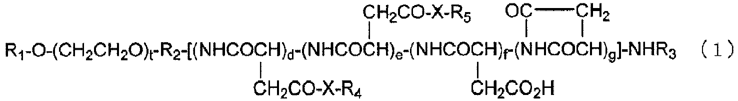 wherein **R**₁ represents a hydrogen atom or a (C1-C6) alkyl group; **R**₂ represents a linking group; **R**₃ represents a hydrogen atom or a (C1-C6) acyl group; **R**₄ represents a residue of an alcoholic hydroxy group of tacrolimus; **R**₅'s are each independently selected from the group consisting of a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group; **X** represents a bond or a linking group; t represents an integer from 5 to 11,500; d represents an integer from 1 to 200; e, f, and g each represent zero or a positive integer of 200 or less; d+e+f+g represents an integer from 3 to 200; and the order of arrangement of the various repeating units of the polyamino acid derivative is arbitrary.
[4] The polymeric derivative of tacrolimus according to [1] or [2], represented by the following General Formula (2): 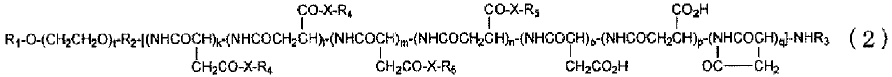 wherein **R**₁, **R**₂, **R**₃, **R**₄, **R**₅, **X,** and t are the same as **R**₁, **R**₂, **R**₃, **R**₄, **R**₅, **X,** and t of General Formula (1), respectively; k, 1, m, n, o, p, and q are each zero or a positive integer of 200 or less; k+l is an integer from 1 to 200; k+l+m+n+o+p+q is an integer from 3 to 200; and the order of arrangement of the various repeating units of the polyamino acid derivative is arbitrary.
[5] The polymeric derivative of tacrolimus according to [3] or [4], wherein **X** represents a bond.
[6] The polymeric derivative of tacrolimus according to [5], wherein **X** represents a bond and **R**₅ represents a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group.
[7] The polymeric derivative of tacrolimus according to [3] or [4], wherein the linking group of **X** is an aspartic acid derivative.
[8] The polymeric derivative of tacrolimus according to [7], wherein the linking group of **X** is an aspartic acid derivative, and **R**₅ represents a hydrophobic substituent and - N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group.
[9] The polymeric compound of tacrolimus according to [3] or [4], wherein the linking group of **X** is represented by the following General Formula (3) or General Formula (4): 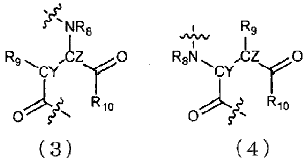 wherein **R**₈ and **R**₉ each independently represent a hydrogen atom or a (C1-C8) alkyl group; **R**₁₀ represents one or more groups selected from the group consisting of NH₂, a linear, branched or cyclic (C1-C20) alkylamino group which may have a substituent, a linear, branched or cyclic (C7-C20) aralkylamino group which may have a substituent, a (C5-C20) aromatic amino group which may have a substituent, and an amino acid residue having a protected carboxy group; and C**Y-**C**Z** represents CH-CH or C=C (double bond).
[10] The polymeric compound of tacrolimus according to [9], wherein **R**₈ and **R**₉ are both a hydrogen atom, and C**Y**-C**Z** is CH-CH.
[11] The polymeric derivative of tacrolimus according to any one of [3] to [10], wherein the hydrophobic substituent is selected from the group consisting of a (C1-C30) alkoxy group, a (C1-C30) alkenyloxy group, a (C1-C30) alkylamino group, a (C2-C60) dialkylamino group, a (C1-C30) alkenylamino group, and an amino acid derivative residue.
[12] The polymeric derivative of tacrolimus according to any one of [3] and [5] to [10], wherein **R**₁ represents a (C1-C6) alkyl group; **R**₂ represents a (C2-C6) alkylene group; **R**₃ represents a (C1-C6) acyl group; t represents an integer from 50 to 1,500; and d+e+f+g represents an integer from 4 to 150.
[13] The polymeric derivative of tacrolimus according to any one of [3] and [5] to [11], wherein **R**₁ represents a (C1-C3) alkyl group; **R**₂ represents (C2-C4) alkylene group; **R**₃ represents a (C1-C3) acyl group; t represents an integer from 100 to 1,500; and d+e+f+g represents an integer from 8 to 120.
[14] The polymeric derivative of tacrolimus according to any one of [4] to [11], wherein **R**₁ represents a (C1-C6) alkyl group; **R**₂ represents a (C2-C6) alkylene group; **R**₃ represents a (C1-C6) acyl group; t represents an integer from 50 to 1,500; and k+l+m+n+o+p+q represents an integer from 4 to 150.
[15] The polymeric derivative of tacrolimus according to any one of [4] to [11], wherein **R**₁ represents a (C1-C3) alkyl group; **R**₂ represents a (C2-C4) alkylene group; **R**₃ represents a (C1-C3) acyl group; t represents an integer from 100 to 1,500; and k+l+m+n+o+p+q represents an integer from 8 to 120.
[16] The polymeric derivative of tacrolimus according to any one of [3] to [15], wherein **R**₁ represents a methyl group; **R**₂ represents a trimethylene group; and **R**₃ represents an acetyl group.
[17] A method for preparing the polymeric derivative of tacrolimus according to any one of [1] to [16], the method including forming an ester-bond between an alcoholic hydroxy group of tacrolimus and a carboxy group in a side chain of a copolymer of a polyethylene glycol segment and a polyaspartic acid derivative by using a dehydration condensing agent in an organic solvent.
[18] A macrolide immunosuppressant including the polymeric derivative of tacrolimus according to any one of [1] to [17] as an active ingredient.

### Advantageous Effects of Invention

The polymeric derivative of tacrolimus of the present invention is characterized by a copolymer of a polyethylene glycol segment and a polyamino acid derivative in which a carboxy group in a side chain of the copolymer is ester-bonded to an alcoholic hydroxy group of tacrolimus. This polymeric derivative may be considered to form, in view of the structure, an aggregate in which the polyethylene glycol segment that is highly hydrophilic in water forms the outer shell, and highly hydrophobic side chains form the inner shell. This polymeric derivative is stable in vivo, is capable of slowly releasing tacrolimus in a non-enzyme-dependent manner, exhibits high accumulability at an inflammation site, and exhibits an excellent therapeutic effect with a low dosage. Furthermore, since the polymeric derivative is capable of non-enzyme-dependent release of a physiologically active substance and maintenance of the blood concentration, control of the dosage depending on the blood kinetics (blood trough concentration) becomes unnecessary, and safety is expected to be markedly enhanced.

### Brief Description of Drawings

Fig. 1 shows the changes over time in drug release in the absence of an enzyme. The symbol -●- represents the compound of Example 1, -○- represents the compound of Example 2, -■- represent the compound of Example 3, and -□- represents the compound of Example 16;
Fig. 2 shows the changes over time in drug release in the absence of an enzyme. The symbol -●- represents the compound of Example 4, -○- represents the compound of Example 5, -■- represent the compound of Example 6, -□- represents the compound of Example 7, and -▲- represents the compound of Example 8;
Fig. 3 shows the changes over time in the tacrolimus concentration in rat blood. The symbol -●- represents the compound of Example 1, -■- represents the compound of Example 2, -Δ- represents the compound of Example 3, -◊- represents the compound of 4, and -*- represents tacrolimus;
Fig. 4 shows the changes over time in the tacrolimus concentration in rat blood. The symbol -●- represents the compound of Example 5, -■- represents the compound of Example 6, -Δ- represents the compound of Example 7, -◊- represents the compound of Example 8, and -*- represents tacrolimus;

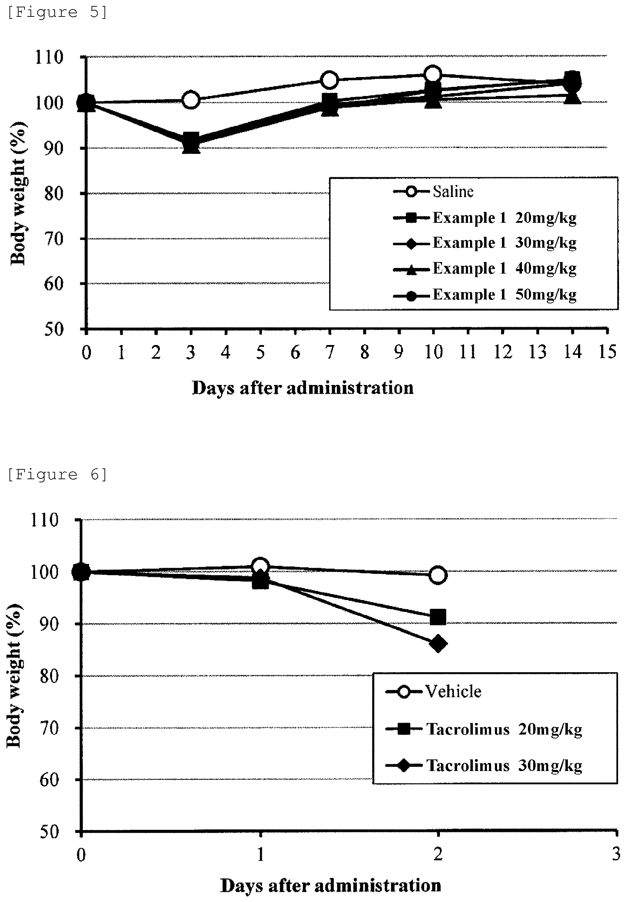

Fig. 5 shows the changes over time in the relative bodyweight of a rat. The symbol -■- represents the bodyweight change occurring when the compound of Example 1 was administered at a dose of 20 mg/kg, -◆- represents the bodyweight change occurring when the compound of Example 1 was administered at a dose of 30 mg/kg, -▲- represents the bodyweight change occurring when the compound of Example 1 was administered at a dose of 40 mg/kg, -●- represents the bodyweight change occurring when the compound of Example 1 was administered at a dose of 50 mg/kg, and -○- represents the bodyweight change occurring when physiological saline was administered once;
Fig. 6 shows the changes over time in the relative bodyweight of a rat. The symbol -■- represents the bodyweight change occurring when tacrolimus was administered at a dose of 20 mg/kg, -◆- represents the bodyweight change occurring when tacrolimus was administered at a dose of 30 mg/kg, and -○- represents the bodyweight change occurring when a mixed liquid of Cremophor and ethanol (vehicle) was administered once;
Fig. 7 shows photographs as drawing substitutes obtained by observing cerebral cortex pathological sections of a rat at various magnification ratios. The left-hand side photographs show pathological sections of a solvent control-administered group, and the right-hand side photographs show pathological sections of an individual that died as a result of tacrolimus administration;
Fig. 8 shows photographs as drawing substitutes showing drug accumulation of the compounds of Examples 9 to 15 in mouse DSS colitis;
Fig. 9 shows the changes over time in the inflammation score of rat collagen-induced arthritis. The symbol -●- represents the compound of Example 1, and -○- represents tacrolimus;
Fig. 10 shows the changes over time in the inflammation score of rat collagen-induced arthritis. The symbol -▲- represents the compound of Example 1, -■- represents the compound of Example 4, and -●- represents the non-administered group;
Fig. 11 shows the changes over time in the inflammation score of rat collagen-induced arthritis. The symbol -▲- represents the compound of Example 1, -■- represents the compound of Example 3, and -●- represents the non-administered group;
Fig. 12 shows the changes over time in the inflammation score of rat collagen-induced arthritis. The symbol -▲- represents the compound of Example 1, -■- represents the compound of Example 2, -◆- represents the compound of Example 5, -*- represents the compound of Example 6, and -●- represents the non-administered group;
Fig. 13 shows the changes over time in the inflammation score of rat collagen-induced arthritis. The symbol -▲- represents the compound of Example 1, -■- represents the compound of Example 7, -◆- represents the compound of Example 8, and -●- represents the non-administered group; and
Fig. 14 shows photographs as drawing substitutes showing drug accumulation of the compound of Synthesis Example 8 in rat collagen-induced arthritis induction. The left-hand side photograph shows a non-arthritis-induced group, and the right-hand side group represents an arthritis-induced group.

### Description of Embodiments

The polymeric derivative of tacrolimus of the present invention is characterized by a copolymer of a polyethylene glycol segment and a polyamino acid derivative in which a carboxy group in a side chain of the copolymer is ester-bonded to an alcoholic hydroxy group of tacrolimus. In the following description, the details will be explained.

The copolymer of a polyethylene glycol segment and a polyamino acid derivative according to the present invention includes a graft-type polymer and a block-type polymer, and a preferred copolymer is a block-type polymer.

The molecular weight of the copolymer of a polyethylene glycol segment and a polyamino acid derivative is usually about 500 to 500,000, preferably about 600 to 100,000, and more preferably 800 to 80,000. Meanwhile, the molecular weight according to the present specification is the peak top molecular weight measured by a GPC method and calculated relative to polyethylene glycol standards.

The number of carboxy groups per molecule of the copolymer of a polyethylene glycol segment and a polyamino acid derivative is, on the average, about 3 to 200, preferably 4 to 150, and more preferably 8 to 120. The number of carboxy groups may be determined by neutralization titration using an alkali.

The polyethylene glycol segment according to the present invention also includes a polyethylene glycol in which both ends are modified or one end is modified, and the modifying groups at both ends may be identical or different. Regarding the terminal modifying group, a (C1-C6) alkyl group which may have a substituent may be mentioned. Specific examples include a methyl group, an ethyl group, a n-propyl group, an i-propyl group, a n-butyl group, a s-butyl group, a t-butyl group, a dimethoxyethyl group, and a diethoxyethyl group. The terminal modifying group is preferably a (C1-C4) alkyl group which may have a substituent; and specific examples include a methyl group, an ethyl group, a n-propyl group, an i-propyl group, a n-butyl group, a s-butyl group, a t-butyl group, and a dimethoxyethyl group. The polyethylene glycol in which one end is modified is preferably an alkoxy polyethylene glycol, and more preferably methoxy polyethylene glycol.

The average molecular weight of the polyethylene glycol segment is usually about 300 to 500,000, preferably about 500 to 100,000, and even more preferably about 1,000 to 50,000.

Examples of the polyamino acid derivative according to the present invention include a polyglutamic acid derivative, a polyaspartic acid derivative, a polylysine derivative, a polyornithine derivative, a polytyrosine derivative, a polyserine derivative, and a polythreonine derivative. The polyamino acid derivative is preferably a polyaspartic acid derivative or a polyglutamic acid derivative, each having a carboxy group as a reactive substituent. These polyamino acid derivatives having side-chain carboxy groups may be an α-amide-bonded type polymer, may be an amide-bonded type polymer with side-chain carboxy groups, may be a β-amide-bonded type polymer, or may be a mixture thereof. The polyamino acid derivative is preferably a polyaspartic acid derivative.

Tacrolimus is represented by the following Formula (I). Tacrolimus has more than one alcoholic hydroxy groups; however, the position of substitution is not limited as long as the hydroxy group is an alcoholic hydroxy group.

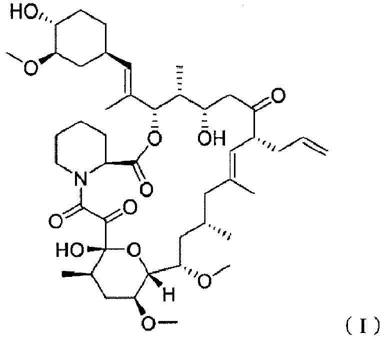

Structural examples of the polymeric derivative of tacrolimus of the present invention include a compound represented by the following General Formula (1), in which **R**₁ represents a hydrogen atom or a (C1-C6) alkyl group; **R**₂ represents a linking group; **R**₃ represents a hydrogen atom or a (C1-C6) acyl group; **R**₄ represents a residue of an alcoholic hydroxy group of tacrolimus; each of **R**₅'s is independently selected from the group consisting of a hydrophobic substituent and -N(**R**₆)CONH(**R**₇), where **R**₆ and **R**₇, which may be identical or different, each represent a (C3-C6) cyclic alkyl group or a (C1-C5) alkyl group which may be substituted with a tertiary amino group; X represents a bond or a linking group; t represents an integer from 5 to 11,500; d, e, f, and g each represent zero or a positive integer of 200 or less; d represents an integer from 1 to 200; and d+e+f+g represents an integer from 3 to 200; and a compound represented by the following General Formula (2), in which t and **R**₁ to **R**₇ have the same definitions as t and **R**₁ to **R**₇ of General Formula (1), respectively; k, 1, m, n, o, p, and q each represents zero or a positive integer of 200 or less; k+l represents an integer from 1 to 200; and k+l+m+n+o+p+q represents an integer from 3 to 200. The order of arrangement of the various repeating units may not be controlled at the time of synthesis, and is not limited to the order of arrangement described in General Formula (1) and General Formula (2).

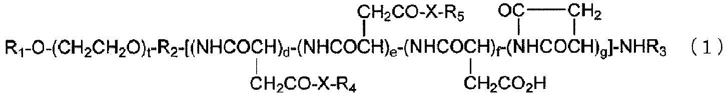

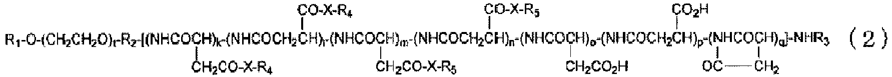

The (C1-C6) alkyl group for **R**₁ of General Formula (1) and General Formula (2) may be a linear or branched (C1-C6) alkyl group, and this alkyl group is preferably a linear or branched (C1-C4) alkyl group, and particularly preferably a linear or branched (C1-C3) alkyl group. Examples of the linear or branched (C1-C6) alkyl group include a methyl group, an ethyl group, a n-propyl group, an i-propyl group, a n-butyl group, and a t-butyl group. Preferred examples include a methyl group, an ethyl group, a n-propyl group, and an i-propyl group, and a methyl group is particularly preferred.

The linking group represented by **R**₂ of General Formula (1) and General Formula (2) is not particularly limited; however, the linking group may be a (C2-C6) alkylene group, and preferably a (C2-C4) alkylene group. Examples include an ethylene group, a trimethylene group, and a butylene group, and a trimethylene group is particularly preferred.

The (C1-C6) acyl group for **R**₃ of General Formula (1) and General Formula (2) is not particularly limited; however, examples include a formyl group, an acetyl group, a propionyl group, and a pivaloyl group, and an acetyl group is particularly preferred.

Regarding the alcoholic hydroxy group of tacrolimus for **R**₄ of General Formula (1) and General Formula (2), the position of substitution is not particularly limited as long as the alcoholic hydroxy group is an alcoholic hydroxy group that forms an ester bond with a carboxylic acid moiety of the polymer by means of a dehydration condensing agent.

A hydrophobic substituent may be adopted for **R**₅ of General Formula (1) and General Formula (2).

Examples of the hydrophobic substituent include a (C1-C30) alkoxy group, a (C1-C30) alkenyloxy group, a (C1-C30) alkylamino group, a (C2-C60) dialkylamino group, a (C1-C30) alkenylamino group, and an amino acid derivative residue.

Particularly preferred examples include a (C1-C20) alkoxy group, a (C1-C20) alkenyloxy group, a (C1-C20) alkylamino group, a (C2-C40) dialkylamino group, a (C1-C20) alkenylamino group, and an amino acid derivative residue.

The (C1-C20) alkoxy group is not particularly limited; however, examples include an octyloxy group, a decyloxy group, a dodecyloxy group, a tetradecyloxy group, a hexadecyloxy group, and an octadecyloxy group.

The (C1-C20) alkenyloxy group is not particularly limited; however, examples include a 9-hexadecenyloxy group, a cis-9-octadecenyloxy group, and a cis,cis-9,12-octadecadienyloxy group.

The (C1-C20) alkylamino group is not particularly limited; however, examples include an octylamino group, a decylamino group, a dodecylamino group, a tetradecylamino group, a hexadecylamino group, and an octadecylamino group.

The (C2-C40) dialkylamino group is not particularly limited; however, examples include a dimethylamino group, a diethylamino group, a dibutylamino group, a dicyclohexylamino group, a dioctylamino group, and a dinonylamino group.

The (C1-C20) alkenylamino group is not particularly limited; however, examples include a 9-hexadecenylamino group, a cis-9-octadecenylamino group, and a cis,cis-9,12-octadecadienylamino group.

Examples of the amino acid derivative residue include a tryptophan derivative residue, a phenylalanine derivative residue, an isoleucine derivative residue, a leucine derivative residue, and a valine derivative residue, and preferred examples include a tryptophan derivative residue, an isoleucine derivative residue, and a phenylalanine derivative residue.

Examples of the tryptophan derivative residue include a tryptophanyl methyl ester group, a tryptophanyl ethyl ester group, a tryptophanyl benzyl ester group, and a tryptophanyl cholesterol ester group, which are represented by the following Formulas (5-1) to (5-4).

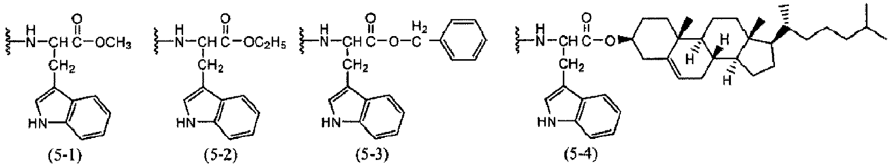

Examples of the isoleucine derivative residue include an isoleucinyl methyl ester group, an isoleucinyl ethyl ester group, an isoleucinyl benzyl ester group, and an isoleucinyl cholesterol ester group, which are represented by the following Formulas (6-1) to (6-4).

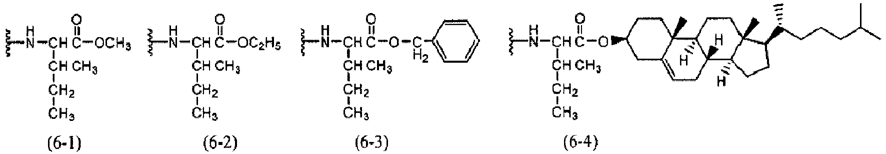

Examples of the phenylalanine derivative residue include a phenylalaninyl methyl ester group, a phenylalaninyl ethyl ester group, a phenylalaninyl benzyl ester group, and a phenylalaninyl cholesterol ester group, which are represented by the following Formulas (7-1) to (7-4).

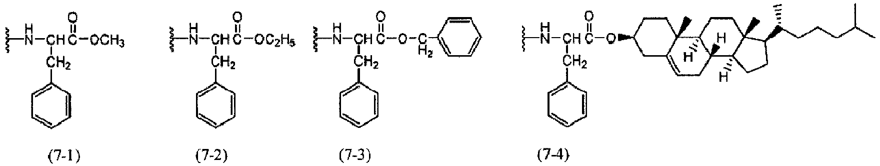

The hydrophobic substituent also includes a fluorescent group, and examples thereof include a 2-(2-aminoethoxy)-9-(diethylamino)-5H-benzo[a]phenoxazin-5-one group, a BODIPY TR Cadaverine residue, an ALEXA FLUOR (registered trademark) 594 Cadaverine residue, a TEXAS RED (registered trademark) Cadaverine residue, and an ATTO 594 amine residue.

**R**₅ of General Formula (1) and General Formula (2) may adopt -N(**R**₆)CONH(**R**₇). Here, **R**₆ and **R**₇ may be identical with or different from each other, and each of them represents a (C3-C6) cyclic alkyl group or a (C1-C5) alkyl group which may be substituted with a tertiary amino group.

Examples of the (C3-C6) cyclic alkyl group include a cyclohexyl group.

Examples of the (C1-C5) alkyl group which may be substituted with a tertiary amino group include an ethyl group, an isopropyl group, and a 3-dimethylaminopropyl group.

Examples of the tertiary amino group of the (C1-C5) alkyl group which may be substituted with a tertiary amino group include a dimethylamino group and a diethylamino group.

The average value of the total number of α-aspartic acid moieties in the polymeric derivative of tacrolimus represented by General Formula (1) is represented by d+e+f+g, and the average value is about 3 to 200, preferably about 6 to 150, and particularly preferably 10 to 120.

The proportion of the number of aspartic acid moieties (d) bonded to tacrolimus with respect to the total number of α-aspartic acid moieties (d+e+f+g) is 1% to 100%, preferably 2% to 90%, and more preferably 3% to 60%. Furthermore, the number of α-aspartic acid moieties (d) is 3 to 200, preferably about 4 to 150, and particularly preferably about 8 to 120.

Examples of preferred combinations of the hydrophobic substituent of **R**₅ with **R**₆ and **R**₇ in -N(**R**₆)CONH(**R**₇) include the compounds disclosed in the following table.

**[Table 1]**

| Combination Example | R₅ (Hydrophobic side chain) | R₆, R₇ |
|---|---|---|
| 1 | Octadecyloxy group | Isopropyl group for both |
| 2 | Octadecyloxy group | One is 3-dimethylaminopropyl group, the other is ethyl group |
| 3 | Octadecyloxy group | Cyclohexyl group for both |
| 4 | Octadecylamino group | Isopropyl group for both |
| 5 | Octadecylamino group | One is 3-dimethylaminopropyl group, the other is ethyl group |
| 6 | Octadecylamino group | Cyclohexyl group for both |
| 7 | Tryptophanyl-benzyl ester group (Formula (5-3)) | Isopropyl group for both |
| 8 | Tryptophanyl-benzyl ester group (Formula (5-3)) | One is 3-dimethylaminopropyl group, the other is ethyl group |
| 9 | Tryptophanyl-benzyl ester group (Formula (5-3)) | Cyclohexyl group for both |
| 10 | Tryptophanyl-cholesterol ester group (Formula (5-4)) | Isopropyl group for both |
| 11 | Tryptophanyl-cholesterol ester group (Formula (5-4)) | One is 3-dimethylaminopropyl group, the other is ethyl group |
| 12 | Tryptophanyl-cholesterol ester group (Formula (5-4)) | Cyclohexyl group for both |
| 13 | Isoleucinyl-benzyl ester group (Formula (6-3)) | Isopropyl group for both |
| 14 | Isoleucinyl-benzyl ester group (Formula (6-3)) | One is 3-dimethylaminopropyl group, the other is ethyl group |
| 15 | Isoleucinyl-benzyl ester group (Formula (6-3)) | Cyclohexyl group for both |
| 16 | Isoleucinyl-cholesterol ester group (Formula (6-4)) | Isopropyl group for both |
| 17 | Isoleucinyl-cholesterol ester group (Formula (6-4)) | One is 3-dimethylaminopropyl group, the other is ethyl group |
| 18 | Isoleucinyl-cholesterol ester group (Formula (6-4)) | Cyclohexyl group for both |
| 19 | Phenylalaninyl-benzyl ester group (Formula (7-3)) | Isopropyl group for both |
| 20 | Phenylalaninyl-benzyl ester group (Formula (7-3)) | One is 3-dimethylaminopropyl group, the other is ethyl group |
| 21 | Phenylalaninyl-benzyl ester group (Formula (7-3)) | Cyclohexyl group for both |
| 22 | Phenylalaninyl-cholesterol ester group (Formula (7-4)) | Isopropyl group for both |
| 23 | Phenylalaninyl-cholesterol ester group (Formula (7-4)) | One is 3-dimethylaminopropyl group, the other is ethyl group |
| 24 | Phenylalaninyl-cholesterol ester group (Formula (7-4)) | Cyclohexyl group for both |

The average value of the total number of aspartic acid moieties in the polymeric derivative of tacrolimus represented by General Formula (2) is represented by k+l+m+n+o+p+q, and the average value is about 3 to 200, preferably about 4 to 150, and particularly preferably 8 to 120.

The proportion of the number of aspartic acid moieties bonded to tacrolimus (k+l) with respect to the total number of aspartic acid moieties (k+l+m+n+o+p+q) in the polymeric derivative of tacrolimus represented by General Formula (2) is 1% to 100%, preferably 2% to 90%, and more preferably 3% to 60%. Furthermore, the number of aspartic acid moieties bonded to tacrolimus (k+l) is 1 to 200, preferably about 1 to 100, and particularly preferably about 1 to 90.

The proportion of α-aspartic acid moieties (k+m+o) with respect to the total number of aspartic acid moieties (k+l+m+n+o+p+q) is 1% to 80%, and preferably 1% to 50%. This proportion may be appropriately changed by, for example, selecting the deprotection conditions for the protected group of polyaspartic acid.

t of General Formula (1) and General Formula (2) is an average value and is an integer from about 5 to 11,500; however, t is preferably an integer from about 50 to 3,000, and particularly preferably an integer from about 100 to 1,500.

**X** in General Formula (1) and General Formula (2) represents a bond or a linking group between **R**₄ or **R**₅ and a side-chain carbonyl group of the polyamino acid main chain. The linking group is not particularly limited as long as the linking group is a linking group having, at the two ends, a functional group that may be bonded to **R**₄ or **R**₅ and a functional group that may be bonded to a side-chain carboxy group of the polyamino acid derivative, respectively.

The functional group of **X** that is bondable to the **R**₄- or **R**₅-side end is preferably a carboxy group, an oxycarboxy group, or an aminocarboxy group. Since **R**₄ and **R**₅ have an amino group and/or a hydroxy group in the molecule, these bondable functional groups form an amide bond, an ester bond, a urethane bond, a carbonate bond, and a urea bond with the amino group and/or hydroxy group.

The other functional group of **X** that is bondable to the side-chain carboxy group-side end is preferably an amino group, a hydroxy group, or a thiol group. These bondable functional groups may form an amide bond, an ester bond, or a thioester bond with a side-chain carboxy group.

That is, **X** is preferably a (C1-C8) alkylene group or alkenylene group which may have a substituent, in which one terminal group is a carboxy group, an oxycarboxy group, or an aminocarboxy group, and the other terminal group is an amino group, a hydroxy group, or a thiol group.

Specific examples of **X** include the groups described in the following table; however, **X** is not limited to these as long as a group that does not affect the synthesis or performance of the polymeric derivative of the present invention is used. **X** should form an amide bond, an ester bond, or a thioester bond with the side-chain carboxy group in all cases.

**[Table 2]**

| Bond with amino group and/or hydroxy group of R₄ or R₅ | X (R₄ or R₅ side → side-chain carboxy group side) |
|---|---|
| Amide bond or ester bond | -CO-(CH₂)_{y}-NH- |
| | -CO-(CH₂)_{y}-O- |
| | -CO-(CH₂)_{y}-S- |
| Urea bond or urethane bond | -CONH-(CH₂)_{y}-NH- |
| | -CONH-(CH₂)_{y}-O- |
| | -CONH-(CH₂)_{y}-S- |
| Carbonate bond | -COO-(CH₂)_{y}-NH- |
| | -COO-(CH₂)_{y}-O- |
| | -COO-(CH₂)_{y}-S- |
| Remark 1) In the table, all of y's represent an integer from 1 to 8. | |
| Remark 2) Hereinafter, X is described as R₄ or R₅ side → side-chain carboxy group side. | |

In the alkylene group of **X**, a hydrogen atom may be modified by an appropriate substituent. Examples of the substituent include a hydroxy group, an amino group, a halogen atom, a (C1-C8) alkyl group, a (C1-C8) alkylcarbonylalkoxy group, a (C1-C8) alkylcarbonylamide group, a (C1-C8) alkylcarbonylalkylamide group, a (C1-C8) alkylaryl group, a (C1-C8) alkoxy group, a (C1-C8) alkylamino group, a (C1-C8) acylamide group, and a (C1-C8) alkoxycarbonylamino group.

**X** is preferably -CO-(CH₂)_{y}-NH- or -CO-(CH₂)_{y}-O-. Particularly preferred is -CO-(CH₂)_{y}-NH- having a carboxy group that may form an amide bond or an ester bond with **R**₄ or **R**₅ and also having an amino group that may form an amide bond with the side-chain carboxy group.

y in **X** is preferably 1 to 6, and more preferably 1, 2, 4, or 6.

The most preferred group for **X** is -CO-(CH₂)_{y}-NH- (where y = 1, 2, 4, or 6).

In regard to -CO-(CH₂)_{y}-NH- that may have a substituent, which has been mentioned as an example of **X**, the case where y is 1 is the same as an amino acid skeleton. Therefore, an amino acid derivative may also be used as **X.**

In the case of using an amino acid derivative as a linking group, the amino acid derivative is used as a linking group in which the N-terminal amino group of the amino acid forms an amide bond with the side-chain carboxy group, and the C-terminal carboxy group forms an amide bond or an ester bond with the amino group or hydroxy group of **R**₄ or **R**₅.

The amino acid that is used as a linking group may be a natural amino acid or a non-natural amino acid, and any of the L-form and the D-form may be used without particular limitations. Examples include hydrocarbon-based amino acids such as glycine, β-alanine, alanine, leucine, and phenylalanine; acidic amino acids such as aspartic acid and glutamic acid; and basic amino acids such as lysine, arginine, and histidine.

The amino acid derivative as **X** is preferably an aspartic acid derivative. The aspartic acid derivative is an aspartic acid derivative linking group in which the α-carboxy group functions as a linking group to **R**₄ or **R**₅, and the β-carboxy group may form an amide form. Alternatively, the aspartic acid derivative may also be an aspartic acid derivative in which the β-carboxy group functions as a linking group to **R**₄ or **R**₅, and the α-carboxy group may form an amide form. In a case in which the other carboxy group, which is not a linking group to **R**₄ or **R**₅, may form an amide form, examples of the amide form include a (C1-C20) alkylamide which may have a substituent, a (C5-C20) aromatic amide which may have a substituent, a (C7-C20) aralkylamide which may have a substituent, and an amino acid residue having a protected carboxy group.

Examples of the (C1-C20) alkylamide which may have a substituent for the aspartic acid derivative include methylamide, ethylamide, isopropylamide, t-butylamide, cyclohexylamide, dodecylamide, and octadecylamide.

Examples of the (C5-C20) aromatic amide which may have a substituent for the aspartic acid derivative include phenylamide, 4-methoxyphenylamide, 4-dimetylaminophenylamide, and 4-hydroxyphenylamide. Examples of the (C7-C20) aralkylamide which may have a substituent for the aspartic acid derivative include benzylamide, 2-phenylethylamide, 4-phenylbutylamide, and 8-phenyloctylamide. Examples of the amino acid amide having a protected carboxy group for the aspartic acid derivative include glycinyl methyl ester, alanyl methyl ester, leucinyl methyl ester, isoleucinyl methyl ester, valinyl methyl ester, phenylalanyl methyl ester, alanyl ethyl ester, leucinyl ethyl ester, isoleucinyl ethyl ester, alanyl butyl ester, and leucinyl butyl ester.

**X** may also adopt an aspartic acid derivative linking group or a maleic acid derivative linking group, which are represented by the following General Formula (3) and General Formula (4), respectively.

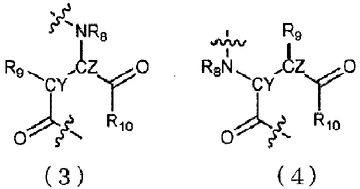

Here, in General Formula (3) and General Formula (4), **R**₈ and **R**₉ each independently represent a hydrogen atom or a (C1-C8) alkylamino group; **R**₁₀ represents one or more groups selected from the group consisting of NH₂, a linear, branched or cyclic (C1-C20) alkylamino group which may have a substituent, a linear, branched or cyclic (C7-C20) aralkylamino group which may have a substituent, a (C5-C20) aromatic amino group which may have a substituent, and an amino acid residue having a protected carboxy group; and C**X-**C**Y** is CH-CH or a C=C (double bond) of **Z**-configuration.

The (C1-C8) alkyl group for **R**₈ or **R**₉ is a linear, branched or cyclic (C1-C8) alkyl group.

Examples of the linear alkyl group include a methyl group, an ethyl group, a n-propyl group, a n-butyl group, and a n-hexyl group.

Examples of the branched alkyl group include an isopropyl group, a t-butyl group, a 1-methylpropyl group, a 2-methylpropyl group, and a 2,2-dimethylpropyl group.

Examples of the cyclic alkyl group include a cyclopropyl group, a cyclobutyl group, a cyclopentyl group, and a cyclohexyl group.

In regard to **R**₁₀, examples of the linear, branched or cyclic (C1-C20) alkylamino group which may have a substituent include a methylamino group, an ethylamino group, an isopropylamino group, a t-butylamino group, a cyclohexylamino group, a n-octylamino group, a dodecylamino group, and an octadecylamino group.

Examples of the linear, branched or cyclic (C7-C20) aralkylamino group which may have a substituent include a benzylamino group, a 2-phenylethylamino group, a 4-phenylbutylamino group, and an 8-phenyloctylamino group.

Examples of the (C5-C20) aromatic amino group which may have a substituent include an anilino group, a 4-methoxyanilino group, a 4-dimethylaminoanilino group, and a 4-hydroxyanilino group.

Furthermore, **R**₁₀ may also be an amino acid residue having a protected carboxy group. Examples of the amino acid residue having a protected carboxy group include a glycinyl methyl ester group represented by the following Formula (8); an alaninyl methyl ester group, an alaninyl ethyl ester group, and an alaninyl butyl ester group, which are represented by the following Formulas (9-1) to (9-3); a leucinyl methyl ester group, a leucinyl ethyl ester group, and a leucinyl butyl ester group, which are represented by the following Formulas (10-1) to (10-3); a valinyl methyl ester group represented by the following Formula (11); and a phenylalaninyl methyl ester group represented by the following Formula (12).

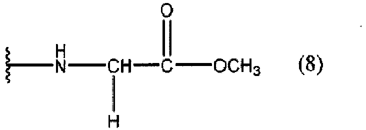

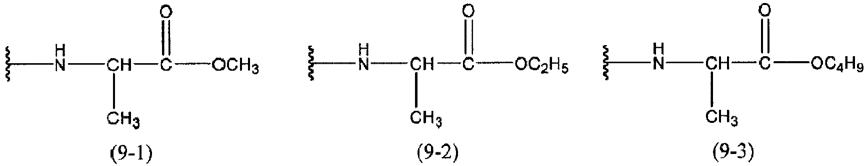

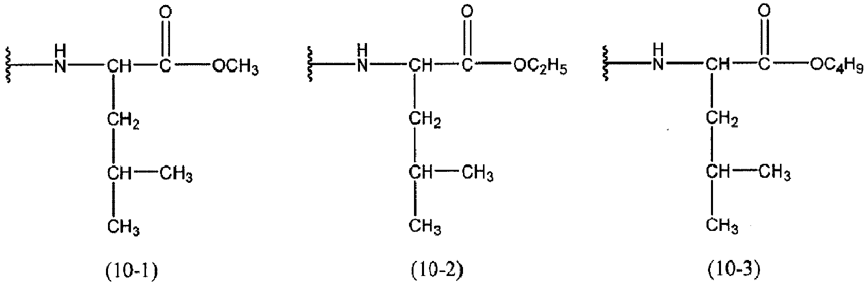

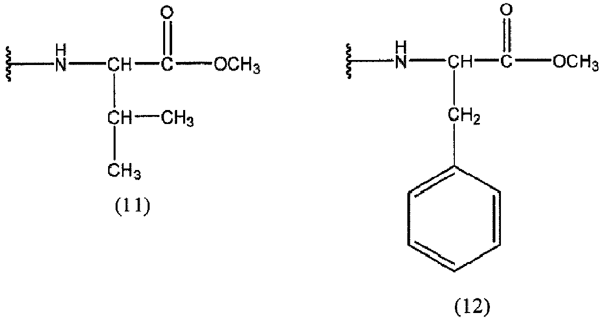

The polymeric derivative of tacrolimus of the present invention is obtained by forming an ester-bond between an alcoholic hydroxy group of tacrolimus and a carboxy group in side chain of a copolymer of a polyethylene glycol segment and a polyamino acid derivative by using a dehydration condensing agent in an organic solvent, and this preparation method is also included in the present invention. That is, the preparation method is a preparation method of subjecting a polyethylene glycol structural moiety-polyaspartic acid block copolymer and tacrolimus, in which functional groups except for a group that will be subjected to a reaction are protected as necessary, to a reaction using a dehydration condensing agent such as dicyclohexylcarbodiimide (DCC), diisopropylcarbodiimide (DIPCI), 1-ethyl-3-(3-dimethylaminopropyl)carbodiimide hydrochloride (EDCI), or 1-ethoxycarbonyl-2-ethoxy-1,2-dihydroxyquinolinone (EEDQ), in an organic solvent that is capable of dissolving the two components, preferably in an aprotic polar solvent such as N,N-dimethylformamide (DMF), 1,3-dimethyl-2-imidazolidinone (DMI), or N-methylpyrrolidone (NMP), at 0°C to 180°C, and preferably 5°C to 50°C. Furthermore, at the time of the condensation reaction, a reaction aid such as N,N-dimethylaminopyridine (DMAP) may also be used. After the condensation reaction, deprotection is performed as necessary, and a polymeric derivative of tacrolimus is produced through conventional operations such as separation and purification.

A polymeric derivative of tacrolimus, in which **R**₅ is a - N(**R**₆)CONH(**R**₇) group, is obtained also by using the above-mentioned carbodiimides as condensing agents.

Regarding a method for introducing a (C1-C30) alkoxy group, a (C1-C30) alkenyloxy group, a (C1-C30) alkylamino group, a (C2-C60) dialkylamino group, a (C1-C30) alkenylamino group, an amino acid derivative residue (including an amino acid having a protected carboxy group), and the like to **R**₅, a method of activating a carboxy group of a polymer by the above-described method, and then reacting the activated carboxy group with a corresponding alcohol, a corresponding amine, an amino acid having a protected carboxy group, or the like in an amount that is wished to be added, under basic conditions; a method activating a corresponding alcohol, a corresponding amine, an amino acid having a protected carboxy group, or the like, and then reacting the activated group with a polymer; and the like are also possible. After a polymer is purified, unreacted carboxy groups in the polymer may be reactivated by a similar reaction, and an alcoholic hydroxy group of tacrolimus may be condensed to one of the reactivated carboxy groups. Alternatively, it is also acceptable that different alcohols, amines and the like are repeatedly reacted, thereby a mixed form of various substituents for **R**₅ is synthesized, and then alcoholic hydroxy groups of tacrolimus may be condensed. Furthermore, it is also acceptable that tacrolimus is condensed, and then a (C1-C30) alkoxy group, a (C1-C30) alkenyloxy group, a (C1-C30) alkylamino group, a (C2-C60) dialkylamino group, a (C1-C30) alkenylamino group, an amino acid derivative residue, and the like may be introduced.

However, the method for preparing the polymeric derivative of tacrolimus of the present invention is not intended to be limited to the above-described methods.

The polymeric derivative of tacrolimus of the present invention has a property of slowly releasing tacrolimus after being administered into the living body, and is offered for use as a medicine containing this tacrolimus as an active ingredient.

The use of the polymeric derivative of tacrolimus of the present invention as a pharmaceutical product is not particularly limited as long as the polymeric derivative is used for a disease for which tacrolimus provides a therapeutic effect. For example, the polymeric derivative of tacrolimus is appropriate for a medicine used for the treatment of autoimmune diseases, inflammatory diseases, allergic diseases, and suppression of rejection in organ transplantation and bone marrow transplantation. Particularly preferably, the polymeric derivative of tacrolimus is a medicine for the treatment of autoimmune diseases or inflammatory diseases. Examples of the autoimmune diseases include rheumatoid arthritis, systemic erythematodes, and ulcerative colitis, and examples of the inflammatory diseases include interstitial pneumonitis.

A medicine containing a polymeric derivative of tacrolimus of the present invention may include other additives that are conventionally acceptable as pharmaceutical products. Examples of the additives include an excipient, an extending agent, a filler, a binder, a wetting agent, a disintegrant, a lubricating agent, a surfactant, a dispersant, a buffering agent, a preservative, a dissolution aid, an antiseptic agent, a flavoring agent, a soothing agent, a stabilizer, and a tonicity adjusting agent.

A medicine containing the polymeric derivative of tacrolimus of the present invention may be prepared as a pharmaceutical preparation for treatment. The preparation may be administered by any administration method such as oral administration, injection, intrarectal administration, portal injection, mixing into the perfusate of an organ, or topical administration into a diseased organ; however, preferably parenteral administration is preferred, and intravenous administration by injection, intraarterial administration, or topical administration into a diseased organ is more preferred. Usually, for example, water, physiological saline, a 5% glucose or mannitol solution, a water-soluble organic solvent (for example, glycerol, ethanol, dimethyl sulfoxide, N-methylpyrrolidone, polyethylene glycol, Cremophor, or a mixed liquid thereof), and a mixed liquid of water and the water-soluble organic solvent are used.

The dosage of the polymeric derivative of tacrolimus of the present invention may be properly changeable depending on the gender, age, physiological conditions, diseased state, and the like of the patient; however, the polymeric derivative of tacrolimus is usually administered parenterally in an amount of 0.01 to 500 mg/m², and preferably 0.1 to 250 mg/m², per day for an adult. Administration by injection is carried out through a vein, an artery, a diseased area (inflammation area), and the like.

The polymeric derivative of tacrolimus of the present invention accumulates in a diseased area, and exhibits a superior effect with a low dosage, compared to simple tacrolimus drug. Furthermore, since non-enzyme-dependent release of a physiologically active substance and maintenance of the blood concentration are enabled, the control of the dosage depending on the blood kinetics (blood trough concentration) becomes unnecessary, and a long interval of administration and safety are markedly enhanced. Therefore, the polymeric derivative of tacrolimus of the present invention is an immunosuppressant or an anti-inflammatory agent useful for the treatment and prevention of rejection to a transplant of an organ or a tissue, a graft-versus-host reaction, an autoimmune disease, an infectious disease, and the like.

### Examples

Hereinafter, the present invention will be further explained by way of Examples. However, the present invention is not intended to be limited to these Examples. Also, the compounds of the present invention were used after being formulated as necessary.

### Synthesis Example 1

### Synthesis of polyethylene glycol-α-polyaspartic acid block copolymer (polyethylene glycol molecular weight: 12,000, degree of polymerization of polyaspartic acid: 20.9) (Compound 1)

A polyethylene glycol having a methoxy group at one end and a 3-aminopropyl group at another end (SUNBRIGHT MEPA-12T, manufactured by NOF Corp., average molecular weight 12 kilodaltons, 100.0 g) was dissolved in DMSO (1,900 mL), subsequently γ-benzyl-L-aspartic acid-N-carboxylic acid anhydride (BLA-NCA, 55.3 g, 27 equivalents) was added to the solution, and the mixture was stirred overnight at 32.5°C. The reaction liquid was added dropwise to a mixed solvent of ethanol (4,000 mL) and diisopropyl ether (16,000 mL) for one hour, and the mixture was stirred for one hour at room temperature. A precipitate was collected by filtration and then dried in a vacuum, and a solid (142.7 g) was obtained. The solid (140.0 g) thus obtained was dissolved in DMF (1,400 mL), acetic anhydride (4.4 mL) was added thereto, and the mixture was stirred for 3 hours at 35°C. The reaction liquid was added dropwise to a mixed solvent of ethanol (1,400 mL) and diisopropyl ether (12,600 mL) for one hour, and the mixture was stirred for one hour at room temperature. A precipitate was collected by filtration and then dried in a vacuum, and a solid (133.7 g) was obtained. The solid (50.0 g) thus obtained was dissolved in DMF (500 mL), subsequently 10% palladium-carbon (5.0 g) was added thereto, and hydrogenolysis was performed for 24 hours at 35°C. Activated carbon (10.0 g) was added to the reaction liquid, the mixture was stirred for one hour, and then the 10% palladium-carbon was separated by filtration. The filtrate was added dropwise to a mixed solvent of ethyl acetate (1,100 mL) and diisopropyl ether (6,000 mL) for one hour, and the mixture was stirred for one hour at room temperature. A precipitate was collected by filtration and then dried in a vacuum, and a solid (41.1 g) was obtained. The solid was dissolved in a 5% saline (2,500 mL), the pH of the solution was adjusted to 11.0 with a 2 N aqueous solution of sodium hydroxide, and then the solution was purified using partition/adsorption resin column chromatography and subsequent ion exchange resin column chromatography. A solution thus eluted was concentrated under reduced pressure and then freeze-dried, and thereby Compound 1 (37.5 g) was obtained. The degree of polymerization of aspartic acid in one molecule of the present compound based on the titration value obtained using a 0.1 N potassium hydroxide solution was about 20.9.

### Synthesis Example 2

### Synthesis of polyethylene glycol-α-polyaspartic acid block copolymer (polyethylene glycol molecular weight: 12,000, degree of polymerization of polyaspartic acid: 40) (Compound 2)

The indicated Compound 2 was obtained by using 51.25 equivalents of γ-benzyl-L-aspartic acid-N-carboxylic acid anhydride for a polyethylene glycol having a methoxy group at one end and a 3-aminopropyl group at another end according to the method described in Synthesis Example 1. The degree of polymerization of aspartic acid in one molecule of the present compound based on the titration value obtained using a 0.1 N potassium hydroxide solution was about 40.8.

### Synthesis Example 3

### Synthesis of aspartic acid-1-glycinyl methyl ester-4-benzyl ester hydrochloride (Compound 3)

N-(t-butoxycarbonyl)aspartic acid-4-benzyl ester (12.01 g) and L-glycine methyl ester hydrochloride (4.65 g) were dissolved in DMF (180 mL), and then 1-ethyl-3-[3-(dimethylamino)propyl]carbodiimide hydrochloride (EDCI) (10.66 g), 1-hydroxybenzotriazole monohydrate (HOBt·H₂O) (6.82 g), and diisopropylethylamine (6.3 mL) were added to the solution. The mixture was stirred for 2.5 hours. Water was added to the reaction liquid, and the mixture was extracted with ethyl acetate. The organic layer was washed with a 5% aqueous solution of citric acid, a saturated aqueous solution of sodium hydrogen carbonate, and saturated brine. The organic layer was dried over sodium sulfate, subsequently ethyl acetate was removed by concentration under reduced pressure, and purification by silica gel column chromatography was performed. The purified product was dried in a vacuum, and thus a white powder (13.79 g) was obtained. This white powder (12.41 g) was dissolved in a 4 N hydrochloric acid-dioxane solution (150 mL), and then the solution was stirred for 4.5 hours at room temperature. The reaction liquid was concentrated under reduced pressure, subsequently ethyl acetate (100 mL) was added thereto, and the mixture was concentrated again under reduced pressure and dried in a vacuum. Thus, Compound 3 (10.58 g) was obtained.

### Synthesis Example 4

### Synthesis of amide conjugate of polyethylene glycol-α-polyaspartic acid block copolymer and aspartic acid-1-glycine methyl ester (polyethylene glycol molecular weight: 12,000, degree of polymerization of polyaspartic acid: 20.9) (Compound 4)

Compound 1 (1.4 g) was dissolved in NMP (25 mL), and Compound 3 (1.0 g), diisopropylethylamine (725 µL), HOBt·H₂O (342 mg), and diisopropylcarbodiimide (DIPCI) (937 µL) were added to the solution at 25°C. The mixture was stirred overnight. The reaction liquid was added dropwise to a mixed solvent of ethanol (40 mL) and diisopropyl ether (160 mL), and the mixture was stirred. A precipitate was collected by filtration and then dried in a vacuum, and a solid (1.87 g) was obtained. The solid (1.7 g) thus obtained was dissolved in NMP (36 mL), subsequently 10% palladium-carbon (180 mg) was added to the solution, and hydrogenolysis was performed overnight at room temperature. Activated carbon (390 mg) was added to the reaction liquid, the mixture was stirred for one hour, and then the 10% palladium-carbon was separated by filtration. The filtrate was added dropwise to a mixed solvent of ethanol (70 mL) and diisopropyl ether (630 mL), and the mixture was stirred. A precipitate was collected by filtration and then dried in a vacuum, and Compound 4 (1.24 g) was obtained.

### Synthesis Example 5

### Synthesis of amide conjugate of polyethylene glycol-α-polyaspartic acid block copolymer and aspartic acid-1-glycine methyl ester (polyethylene glycol molecular weight: 12,000, degree of polymerization of polyaspartic acid: 40.8) (Compound 5)

Compound 2 (1.5 g) was dissolved in NMP (27 mL), and Compound 3 (1.8 g), diisopropylethylamine (978 µL), HOBt·H₂O (671 mg), and DIPCI (1.12 mL) were added to the solution at 25°C. The mixture was stirred overnight. The reaction liquid was added dropwise to a mixed solvent of ethanol (75 mL) and diisopropyl ether (300 mL), and the mixture was stirred. A precipitate was collected by filtration and the dried in a vacuum, and a solid (2.3 g) was obtained. The solid (2.3 g) thus obtained was dissolved in NMP (35 mL), and then 10% palladium-carbon (400 mg) was added thereto. Hydrogenolysis was performed overnight at room temperature. Activated carbon (800 mg) was added to the reaction liquid, the mixture was stirred for one hour, and then the 10% palladium-carbon was separated by filtration. The filtrate was added dropwise to diisopropyl ether (500 mL), and the mixture was stirred. A precipitate was collected by filtration and then dried in a vacuum, and Compound 5 (2.26 g) was obtained.

### Synthesis Example 6

### Synthesis of polyethylene glycol-α,β-polyaspartic acid block copolymer (polyethylene glycol molecular weight: 12,000, degree of polymerization of polyaspartic acid: 23.8) (Compound 6)

A polyethylene glycol having a methoxy group at one end and a 3-aminopropyl group at another end (SUNBRIGHT MEPA-12T, manufactured by NOF Corp., average molecular weight 12 kilodaltons, 75 g) was dissolved in DMSO (1.43 L), and then γ-benzyl-L-aspartic acid-N-carboxylic acid anhydride (BLA-NCA, 45 g, 29 equivalents) was added to the solution. The mixture was stirred overnight at 32.0°C. The reaction liquid was added dropwise to a mixed solvent of ethanol (3 L) and diisopropyl ether (12 L) for one hour, and the mixture was stirred for one hour at room temperature. A precipitate was collected by filtration and then dried in a vacuum, and a solid (106 g) was obtained. The solid (105 g) thus obtained was dissolved in DMF (1.05 L), acetic anhydride (3.3 mL) was added thereto, and the mixture was stirred for 3 hours at 35°C. The reaction liquid was added dropwise to a mixed solvent of ethanol (1.05 L) and diisopropyl ether (9.45 L) for one hour, and the mixture was stirred for one hour at room temperature. A precipitate was collected by filtration and then dried in a vacuum, and a solid (103 g) was obtained. The solid (100 g) thus obtained was dissolved in MeCN (2 L), subsequently a 0.2 N sodium hydroxide solution (2 L) was added to the solution, and hydrolysis was performed for 3 hours at 23°C. 2 N hydrochloric acid was added to the reaction liquid to neutralize the reaction liquid, and then acetonitrile was removed by concentration under reduced pressure to obtain a concentrated liquid. The concentrated liquid was washed three times using ethyl acetate (2 L). The aqueous layer was concentrated under reduced pressure, and then the pH of the solution was adjusted to 11.0 with a 1 N aqueous solution of sodium hydroxide. Sodium chloride (100 g) was added thereto, and then the mixture was purified using partition/adsorption resin column chromatography and subsequent ion exchange resin column chromatography. A solution thus eluted was concentrated under reduced pressure and then freeze-dried, and Compound 6 (75.4 g) was obtained. The degree of polymerization of aspartic acid in one molecule of the present compound based on the titration value obtained using a 0.1 N potassium hydroxide solution was 23.8.

### Synthesis Example 7

### Synthesis of amide conjugate of polyethylene glycol-α,β-polyaspartic acid block copolymer and aspartic acid-1-glycine methyl ester (polyethylene glycol molecular weight: 12,000, degree of polymerization of polyaspartic acid: 23.8) (Compound 7)

Compound 6 (1.24 g) was dissolved in DMF (20 mL), and Compound 3 (992 mg), diisopropylethylamine (536 µL), HOBt·H₂O (30.6 mg), and DIPCI (616 µL) were added to the solution at 25°C. The mixture was stirred overnight. The reaction liquid was added dropwise to a mixed solvent of ethanol (40 mL) and diisopropyl ether (160 mL), and the mixture was stirred. A precipitate was collected by filtration and then dried in a vacuum, and a solid (1.88 g) was obtained. The solid (1.75 g) thus obtained was dissolved in DMF (35 mL), subsequently 10% palladium-carbon (175 mg) was added thereto, and hydrogenolysis was performed overnight at room temperature. Activated carbon (384 mg) was added to the reaction liquid, the mixture was stirred for 2 hours, and then the 10% palladium-carbon was separated by filtration. The filtrate was added dropwise to a mixed solvent of ethanol (45 mL) and diisopropyl ether (405 mL), and the mixture was stirred. A precipitate was collected by filtration and then dried in a vacuum, and Compound 7 (1.44 g) was obtained.

### Example 1

Polymeric derivative in which in regard to General Formula (2), **R**₁ is methyl group, **R**₂ is trimethylene group, **R**₃ is acetyl group, **R**₄ is tacrolimus, **R**₅ is isopropylaminocarbonylisopropylamino group, **X** is a bond, average value of t is 272, average value of k+l+m+n+o+p+q is 23.8, and average value of k+l is 5.3 **(Compound 8)**

Compound 6 (832 mg) and tacrolimus (540 mg) were dissolved in DMF (8.9 mL), and DMAP (16.4 mg) and DIPCI (474 µL) were added to the solution at 15°C. The mixture was stirred overnight. The reaction liquid was added dropwise to diisopropyl ether (270 mL), and the mixture was stirred. A precipitate was collected by filtration and dried in a vacuum, and a solid thus obtained was dissolved in acetonitrile (28 mL). Subsequently, purified water (28 mL) and an ion exchange resin (14 mL) were added to the solution, the mixture was stirred for 3 hours, and then the ion exchange resin was separated by filtration. The filtrate was concentrated under reduced pressure and then freeze-dried, and thereby Compound 8 (980 mg) was obtained. The tacrolimus content of Compound 8 was calculated to be 20.8%.

### Example 2

Polymeric derivative in which in regard to General Formula (2), **R**₁ is methyl group, **R**₂ is trimethylene group, **R**₃ is acetyl group, **R**₄ is tacrolimus, **R**₅ is tryptophanyl cholesterol ester group (Formula (5-4)) and isopropylaminocarbonylisopropylamino group, **X** is a bond, average value of t is 272, and average value of k+l+m+n+o+p+q is 23.8 **(Compound 9)**

N-(tert-butoxycarbonyl)-L-tryptophan (3.54 g) and cholesterol (3.0 g) were dissolved in dichloromethane (38.8 mL), and DMAP (948.0 mg) and EDCI (793.3 mg) were added to the solution. The mixture was stirred overnight. A saturated aqueous solution of ammonium chloride was added to the reaction liquid, and the mixture was extracted with dichloromethane. The organic layer was dried over sodium sulfate, and then dichloromethane was removed by concentration under reduced pressure. Subsequently, the residue was purified by silica gel column chromatography and dried in a vacuum, and thus a white powder (3.13 g) was obtained. This white powder (3.13 g) was dissolved in a 4 N hydrochloric acid-dioxane solution (23.25 mL), and then the solution was stirred for 3 hours at room temperature. Acetone was added to the reaction liquid to precipitate a solid, and then the precipitate was collected by filtration and dried in a vacuum. Thus, a white powder (2.69 g) was obtained.

This white powder (60.6 mg), Compound 6 (308 mg), and tacrolimus (200.0 mg) were dissolved in NMP (3.3 mL), and diisopropylethylamine (25.4 µL), DMAP (30.4 mg), and DIPCI (153 µL) were added to the solution at 25°C. The mixture was stirred overnight. The reaction liquid was added dropwise to diisopropyl ether (100 mL), and the mixture was stirred. A precipitate was collected by filtration and dried in a vacuum, and a solid thus obtained was dissolved in acetonitrile (10.4 mL). Subsequently, purified water (10.4 mL) and an ion exchange resin (5.2 mL) were added to the solution, the mixture was stirred for 2.5 hours, and then the ion exchange resin was separated by filtration. The filtrate was concentrated under reduced pressure and then freeze-dried, and thereby Compound 9 (420 mg) was obtained. The tacrolimus content of Compound 9 was calculated to be 19.3%.

### Example 3

Polymeric derivative in which in regard to General Formula (2), **R**₁ is methyl group, **R**₂ is trimethylene group, **R**₃ is acetyl group, **R**₄ is tacrolimus, **R**₅ is isopropylaminocarbonylisopropylamino group, **X** is represented by General Formula (3), **R**₈ and **R**₉ are hydrogen atoms, C**Y**-C**Z** is CH-CH, **R**₁₀ is glycinyl methyl ester group, average value of t is 272, and average value of k+l+m+n+o+p+q is 23.8 **(Compound 10)**

Compound 7 (339 mg), tacrolimus (150 mg), and DMAP (22.3 mg) were dissolved in NMP (2.4 mL), and DIPCI (129 µL) was added to the solution at 25°C. The mixture was stirred overnight. The reaction liquid was added dropwise to diisopropyl ether (75 mL), and the mixture was stirred. A precipitate was collected by filtration and dried in a vacuum, and a solid thus obtained was dissolved in acetonitrile (10 mL). Subsequently, purified water (10 mL) and an ion exchange resin (5 mL) were added to the solution, the mixture was stirred for one hour, and then the ion exchange resin was separated by filtration. The filtrate was concentrated under reduced pressure and then freeze-dried, and thereby Compound 10 (349 mg) was obtained. The tacrolimus content of Compound 10 was calculated to be 22.5%.

### Example 4

Polymeric derivative in which in regard to General Formula (1), **R**₁ is methyl group, **R**₂ is trimethylene group, **R**₃ is acetyl group, **R**₄ is tacrolimus, **R**₅ is isopropylaminocarbonylisopropylamino group, **X** is a bond, average value of t is 272, and average value of d+e+f+g is 20.9 **(Compound 11)**

Compound 1 (588 mg) and tacrolimus (307 mg) were dissolved in DMF (8.5 mL), and N,N-dimethylaminopyridine (DMAP) (9.1 mg) and DIPCI (260 µL) were added to the solution at 25°C. The mixture was stirred overnight. The reaction liquid was added dropwise to diisopropyl ether (255 mL), and the mixture was stirred. A precipitate was collected by filtration and dried in a vacuum, and a solid thus obtained was dissolved in acetonitrile (24 mL). Subsequently, purified water (24 mL) and an ion exchange resin (12 mL) were added to the solution, the mixture was stirred for 3 hours, and then the ion exchange resin was separated by filtration. The filtrate was concentrated under reduced pressure and then freeze-dried, and thereby Compound 11 (623 mg) was obtained. The tacrolimus content of Compound 11 was calculated to be 11.2%.

### Example 5

Polymeric derivative in which in regard to General Formula (1), **R**₁ is methyl group, **R**₂ is trimethylene group, **R**₃ is acetyl group, **R**₄ is tacrolimus, **R**₅ is octadecylamino group and isopropylaminocarbonylisopropylamino group, **X** is a bond, average value of t is 272, and average value of d+e+f+g is 20.9 **(Compound 12)**

Compound 1 (516 mg), tacrolimus (300 mg), and octadecylamine (40 mg) were dissolved in NMP (5 mL), and DMAP (46 mg) and DIPCI (230 µL) were added to the solution at 30°C. The mixture was stirred overnight. The reaction liquid was added dropwise to diisopropyl ether (155 mL), and the mixture was stirred. A precipitate was collected by filtration and dried in a vacuum, and a solid thus obtained was dissolved in acetonitrile (17 mL). Subsequently, purified water (17 mL) and an ion exchange resin (8.7 mL) were added to the solution, the mixture was stirred for 3 hours, and then the ion exchange resin was separated by filtration. The filtrate was concentrated under reduced pressure and then freeze-dried, and thereby Compound 12 (620 mg) was obtained. The tacrolimus content of Compound 12 was calculated to be 14.1%.

### Example 6

Polymeric derivative in which in regard to General Formula (1), **R**₁ is methyl group, **R**₂ is trimethylene group, **R**₃ is acetyl group, **R**₄ is tacrolimus, **R**₅ is octadecylamino group and isopropylaminocarbonylisopropylamino group, **X** is a bond, average value of t is 272, and average value of d+e+f+g is 40.8 **(Compound 13)**

Compound 2 (337 mg), tacrolimus (330 mg), and octadecylamine (44 mg) were dissolved in NMP (5.5 mL), and DMAP (50 mg) and DIPCI (253 µL) were added to the solution at 25°C. The mixture was stirred overnight. The reaction liquid was added dropwise to diisopropyl ether (165 mL), and the mixture was stirred. A precipitate was collected by filtration and dried in a vacuum, and a solid thus obtained was dissolved in acetonitrile (17 mL). Subsequently, purified water (17 mL) and an ion exchange resin (8.7 mL) were added thereto, the mixture was stirred for 3 hours, and then the ion exchange resin was separated by filtration. The filtrate was concentrated under reduced pressure and then freeze-dried, and thereby Compound 13 (507 mg) was obtained. The tacrolimus content of Compound 13 was calculated to be 23.6%.

### Example 7

Polymeric derivative in which in regard to General Formula (1), **R**₁ is methyl group, **R**₂ is trimethylene group, **R**₃ is acetyl group, **R**₄ is tacrolimus, **R**₅ is octadecylamino group and isopropylaminocarbonylisopropylamino group, **X** is represented by General Formula (3), **R**₈ and **R**₉ are hydrogen atoms, C**Y**-C**Z** is CH-CH, **R**₁₀ is glycinyl methyl ester group, average value of t is 272, and average value of d+e+f+g is 20.9 **(Compound 14)**

Compound 4 (849 mg), tacrolimus (515 mg), octadecylamine (47.4 mg), and DMAP (52.1 mg) were dissolved in NMP (6 mL), and DIPCI (657 µL) was added to the solution at 35°C. The mixture was stirred two nights. The reaction liquid was added dropwise to diisopropyl ether (200 mL), and the mixture was stirred. A precipitate was collected by filtration and then dried in a vacuum, and a solid (886 mg) was obtained. The solid thus obtained was dissolved in acetonitrile (30 mL), and then purified water (30 mL) and an ion exchange resin (18 mL) were added to the solution. The mixture was stirred for one hour, and then the ion exchange resin was separated by filtration. The filtrate was concentrated under reduced pressure and freeze-dried, and thereby Compound 14 (0.87 g) was obtained. The tacrolimus content of Compound 14 was calculated to be 15.7%.

### Example 8

Polymeric derivative in which in regard to General Formula (1), **R**₁ is methyl group, **R**₂ is trimethylene group, **R**₃ is acetyl group, **R**₄ is tacrolimus, **R**₅ is octadecylamino group and isopropylaminocarbonylisopropylamino group, **X** is represented by General Formula (3), **R**₈ and **R**₉ are hydrogen atoms, C**Y**-C**Z** is CH-CH, **R**₁₀ is glycinyl methyl ester group, average value of t is 272, and average value of d+e+f+g is 40.8 **(Compound 15)**

Compound 5 (357 mg), tacrolimus (330 mg), octadecylamine (29.5 mg), and DMAP (33.4 mg) were dissolved in NMP (4 mL), and DIPCI (168 µL) was added to the solution at 35°C. The mixture was stirred two nights. The reaction liquid was added dropwise to diisopropyl ether (120 mL), and the mixture was stirred. A precipitate was collected by filtration and dried in a vacuum, and a solid thus obtained was dissolved in acetonitrile (11 mL). Subsequently, purified water (11 mL) and an ion exchange resin (5.5 mL) were added to the solution, the mixture was stirred for 3 hours, and then the ion exchange resin was separated by filtration. The filtrate was concentrated under reduced pressure and then freeze-dried, and thereby Compound 15 (424 mg) was obtained. The tacrolimus content of Compound 15 was calculated to be 17.4%.

### Example 9

Polymeric derivative in which in regard to General Formula (2), **R**₁ is methyl group, **R**₂ is trimethylene, **R**₃ is acetyl group, **R**₄ is tacrolimus, **R**₅ is 2-(2-aminoethoxy)-9-(diethylamino)-5H-benzo[a]phenoxazin-5-one residue and isopropylaminocarbonylisopropylamino group, **X** is a bond, average value of t is 272, and average value of k+l+m+n+o+p+q is 23.8 **(Compound 16)**

Compound 6 (524.7 mg), tacrolimus (340.7 mg), and 2-(2-aminoethoxy)-9-(diethylamino)-5H-benzo[a]phenoxazin-5-one (manufactured by Fuji Molecular Planning Co., Ltd.) (8 mg) were dissolved in DMF (5.7 mL), and DMAP (10.4 mg) and DIPCI (301.8 µL) were added to the solution at 15°C. The mixture was stirred overnight. The reaction liquid was added dropwise to diisopropyl ether (170 mL), and the mixture was stirred. A precipitate was collected by filtration and dried in a vacuum, and a solid thus obtained was dissolved in acetonitrile (10 mL). Subsequently, purified water (10 mL) and an ion exchange resin (16 mL) were added to the solution, the mixture was stirred for 3 hours, and then the ion exchange resin was separated by filtration. The filtrate was concentrated under reduced pressure and then freeze-dried, and thereby Compound 16 (627 mg) was obtained. The tacrolimus content of Compound 16 was calculated to be 24.8%, and the content of 2-(2-aminoethoxy)-9-(diethylamino)-5H-benzo[a]phenoxazin-5-one was calculated to be 1.2%.

### Example 10

Polymeric derivative in which in regard to General Formula (2), **R**₁ is methyl group, **R**₂ is trimethylene, **R**₃ is acetyl group, **R**₄ is tacrolimus, **R**₅ is tryptophanyl cholesterol ester group (Formula (5-4)), 2-(2-aminoethoxy)-9-(diethylamino)-5H-benzo[a]phenoxazin-5-one residue, and isopropylaminocarbonylisopropylamino group, **X** is a bond, average value of t is 272, and average value of k+l+m+n+o+p+q is 23.8 **(Compound 17)**

N-(tert-butoxycarbonyl)-L-tryptophan (3.54 g) and cholesterol (3.0 g) were dissolved in dichloromethane (38.8 mL), and DMAP (948.0 mg) and EDCI (793.3 mg) were added to the solution. The mixture was stirred overnight. A saturated aqueous solution of ammonium chloride was added to the reaction liquid, and the mixture was extracted with dichloromethane. The organic layer was dried over sodium sulfate, and then dichloromethane was removed by concentration under reduced pressure. Subsequently, the residue was purified by silica gel column chromatography and dried in a vacuum, and thus a white powder (3.13 g) was obtained. This white powder (3.13 g) was dissolved in a 4 N hydrochloric acid-dioxane solution (23.25 mL), and then the solution was stirred for 3 hours at room temperature. Acetone was added to the reaction liquid to precipitate a solid, and then the precipitate was collected by filtration and dried in a vacuum. Thus, a white powder (2.69 g) was obtained.

This white powder (32.3 mg), Compound 6 (163.8 mg), tacrolimus (106.4 mg), and 2-(2-aminoethoxy)-9-(diethylamino)-5H-benzo[a]phenoxazin-5-one (manufactured by Fuji Molecular Planning Co., Ltd.) (1.5 mg) were dissolved in NMP (1.8 mL), and diisopropylethylamine (13.5 µL), DMAP (16.2 mg), and DIPCI (81.6 µL) were added to the solution at 30°C. The mixture was stirred overnight. The reaction liquid was added dropwise to diisopropyl ether (50 mL), and the mixture was stirred. A precipitate was collected by filtration and dried in a vacuum, and a solid thus obtained was dissolved in acetonitrile (6 mL). Subsequently, purified water (6 mL) and an ion exchange resin (3 mL) were added to the solution, the mixture was stirred for 4 hours, and then the ion exchange resin was separated by filtration. The filtrate was concentrated under reduced pressure and then freeze-dried, and thereby Compound 17 was obtained. The tacrolimus content of Compound 17 was calculated to be 15.3%, and the content of 2-(2-aminoethoxy)-9-(diethylamino)-5H-benzo[a]phenoxazin-5-one was calculated to be 0.59%.

### Example 11

Polymeric derivative in which in regard to General Formula (2), **R**₁ is methyl group, **R**₂ is trimethylene group, **R**₃ is acetyl group, **R**₄ is tacrolimus, **R**₅ is 2-(2-aminoethoxy)-9-(diethylamino)-5H-benzo[a]phenoxazin-5-one residue and isopropylaminocarbonylisopropylamino group, **X** is represented by General Formula (3), **R**₈ and **R**₉ are hydrogen atoms, C**Y**-C**Z** is CH-CH, **R**₁₀ is glycinyl methyl ester group, average value of t is 272, and average value of k+l+m+n+o+p+q is 23.8

### (Compound 18)

Compound 7 (197 mg), tacrolimus (85.2 mg), 2-(2-aminoethoxy)-9-(diethylamino)-5H-benzo[a]phenoxazin-5-one (manufactured by Fuji Molecular Planning Co., Ltd.) (2 mg), and DMAP (12.9 mg) were dissolved in NMP (1.4 mL), and DIPCI (75 µL) were added to the solution at 25°C. The mixture was stirred overnight. The reaction liquid was added dropwise to diisopropyl ether (40 mL), and the mixture was stirred. A precipitate was collected by filtration and dried in a vacuum, and a solid thus obtained was dissolved in acetonitrile (6 mL). Subsequently, purified water (6 mL) and an ion exchange resin (3 mL) were added to the solution, the mixture was stirred for one hour, and then the ion exchange resin was separated by filtration. The filtrate was concentrated under reduced pressure and then freeze-dried, and thereby Compound 18 (132 mg) was obtained. The tacrolimus content of Compound 17 was calculated to be 19.8%, and the content of 2-(2-aminoethoxy)-9-(diethylamino)-5H-benzo[a]phenoxazin-5-one was calculated to be 0.74%.

### Example 12

Polymeric derivative in which in regard to General Formula (1), **R**₁ is methyl group, **R**₂ is trimethylene, **R**₃ is acetyl group, **R**₄ is tacrolimus, **R**₅ is 2-(2-aminoethoxy)-9-(diethylamino)-5H-benzo[a]phenoxazin-5-one residue and isopropylaminocarbonylisopropylamino group, **X** is a bond, average value of t is 272, and average value of d+e+f+g is 20.3 **(Compound 19)**

Compound 1 (224 mg), tacrolimus (117 mg), and 2-(2-aminoethoxy)-9-(diethylamino)-5H-benzo[a]phenoxazin-5-one (manufactured by Fuji Molecular Planning Co., Ltd.) (1.5 mg) were dissolved in DMF (3.6 mL), and DMAP (3.5 mg) and DIPCI (101 µL) were added to the solution at 25°C. The mixture was stirred overnight. The reaction liquid was added dropwise to diisopropyl ether (108 mL), and the mixture was stirred. A precipitate was collected by filtration and dried in a vacuum, and a solid thus obtained was dissolved in acetonitrile (12 mL). Subsequently, purified water (12 mL) and an ion exchange resin (6 mL) were added to the solution, the mixture was stirred for 3 hours, and then the ion exchange resin was separated by filtration. The filtrate was concentrated under reduced pressure and then freeze-dried, and thereby Compound 19 (246 mg) was obtained. The tacrolimus content of Compound 19 was calculated to be 9.3%, and the content of 2-(2-aminoethoxy)-9-(diethylamino)-5H-benzo[a]phenoxazin-5-one was calculated to be 0.51%.

### Example 13

Polymeric derivative in which in regard to General Formula (1), **R**₁ is methyl group, **R**₂ is trimethylene, **R**₃ is acetyl group, **R**₄ is tacrolimus, **R**₅ is octadecylamino group, 2-(2-aminoethoxy)-9-(diethylamino)-5H-benzo[a]phenoxazin-5-one residue, and isopropylaminocarbonylisopropylamino group, **X** is a bond, average value of t is 272, and average value of d+e+f+g is 20.9 **(Compound 20)**

Compound 1 (157 mg), tacrolimus (91 mg), octadecylamine (12 mg), and 2-(2-aminoethoxy)-9-(diethylamino)-5H-benzo[a]phenoxazin-5-one (manufactured by Fuji Molecular Planning Co., Ltd.) (1.5 mg) were dissolved in NMP (1.5 mL), and DMAP (14 mg) and DIPCI (70 µL) were added to the solution at 30°C. The mixture was stirred overnight. The reaction liquid was added dropwise to diisopropyl ether (45 mL), and the mixture was stirred. A precipitate was collected by filtration and dried in a vacuum, and a solid thus obtained was dissolved in acetonitrile (5.2 mL). Subsequently, purified water (5.2 mL) and an ion exchange resin (2.6 mL) were added to the solution, the mixture was stirred for 3 hours, and then the ion exchange resin was separated by filtration. The filtrate was concentrated under reduced pressure and then freeze-dried, and thereby Compound 20 (192 mg) was obtained. The tacrolimus content of Compound 20 was calculated to be 13.2%, and the content of 2-(2-aminoethoxy)-9-(diethylamino)-5H-benzo[a]phenoxazin-5-one was calculated to be 0.70%.

### Example 14

Polymeric derivative in which in regard to General Formula (1), **R**₁ is methyl group, **R**₂ is trimethylene, **R**₃ is acetyl group, **R**₄ is tacrolimus, **R**₅ is octadecylamino group, 2-(2-aminoethoxy)-9-(diethylamino)-5H-benzo[a]phenoxazin-5-one residue, and isopropylaminocarbonylisopropylamino group, **X** is a bond, average value of t is 272, and average value of d+e+f+g is 40.8 **(Compound 21)**

Compound 2 (99 mg), tacrolimus (97 mg), octadecylamine (13 mg), and 2-(2-aminoethoxy)-9-(diethylamino)-5H-benzo[a]phenoxazin-5-one (manufactured by Fuji Molecular Planning Co., Ltd.) (1.5 mg) were dissolved in NMP (1.6 mL), and DMAP (15 mg) and DIPCI (74 µL) were added to the solution at 25°C. The mixture was stirred overnight. The reaction liquid was added dropwise to diisopropyl ether (48 mL), and the mixture was stirred. A precipitate was collected by filtration and dried in a vacuum, and a solid thus obtained was dissolved in acetonitrile (5 mL). Subsequently, purified water (5 mL) and an ion exchange resin (2.5 mL) were added to the solution, the mixture was stirred for 3 hours, and then the ion exchange resin was separated by filtration. The filtrate was concentrated under reduced pressure and then freeze-dried, and thereby Compound 21 (141 mg) was obtained. The tacrolimus content of Compound 21 was calculated to be 24.8%, and the content of 2-(2-aminoethoxy)-9-(diethylamino)-5H-benzo[a]phenoxazin-5-one was calculated to be 0.89%.

### Example 15

Polymeric derivative in which in regard to General Formula (1), **R**₁ is methyl group, **R**₂ is trimethylene, **R**₃ is acetyl group, **R**₄ is tacrolimus, **R**₅ is octadecylamino group, 2-(2-aminoethoxy)-9-(diethylamino)-5H-benzo[a]phenoxazin-5-one residue, and isopropylaminocarbonylisopropylamino group, **X** is linking group having a structure of General Formula (3), **R**₈ and **R**₉ are hydrogen atoms, C**Y**-C**Z** is CH-CH, **R**₁₀ is glycinyl methyl ester group, average value of t is 272, and average value of d+e+f+g is 20.9 **(Compound 22)**

Compound 4 (302 mg), tacrolimus (181 mg), octadecylamine (16.8 mg), 2-(2-aminoethoxy)-9-(diethylamino)-5H-benzo[a]phenoxazin-5-one (manufactured by Fuji Molecular Planning Co., Ltd.) (1.96 mg), and DMAP (18.4 mg) were dissolved in NMP (2 mL), and DIPCI (93.4 µL) was added to the solution at 35°C. The mixture was stirred two nights. The reaction liquid was added dropwise to diisopropyl ether (64 mL), and the mixture was stirred. A precipitate was collected by filtration and then dried in a vacuum, and a solid (316 mg) was obtained. The solid thus obtained was dissolved in acetonitrile (10 mL), and then purified water (10 mL) and an ion exchange resin (6 mL) were added to the solution. The mixture was stirred for 0.5 hours, and then the ion exchange resin was separated by filtration. The filtrate was concentrated under reduced pressure and then freeze-dried, and thereby Compound 22 (282 mg) was obtained. The tacrolimus content of Compound 22 was calculated to be 17.6%, and the content of 2-(2-aminoethoxy)-9-(diethylamino)-5H-benzo[a]phenoxazin-5-one was calculated to be 0.46%.

### Example 16

### Polymeric derivative of PEG-pGlu-Ac and tacrolimus (Compound 23)

DMF (3 mL) and tacrolimus (85 mg) were added to PEG-pGlu-Ac (PEG: average molecular weight 12,000; polyglutamic acid: average degree of polymerization 22) (0.28 g) produced by the method described in Japanese Patent No. 4745664, and the mixture was dissolved at 35°C. DMAP (5.1 mg) and DIPCI (0.15 mL) were added to the solution at 15°C, and the mixture was stirred overnight. After 21.5 hours, DIPCI (0.074 mL) was added thereto, the reaction temperature was raised to 25°C, and the mixture was stirred for 3 hours. The reaction liquid was added dropwise to diisopropyl ether (30 mL) for 10 minutes, and the mixture was stirred for 2 hours at room temperature. A precipitate was collected by filtration and washed with diisopropyl ether, and then a precipitate thus obtained was dissolved in acetonitrile (10 mL). Subsequently, purified water (10 mL) and an ion exchange resin (DOWEX 50 (H⁺) manufactured by Dow Chemical Company, 5 mL) were added to the solution. After the mixture was stirred for 3 hours, the ion exchange resin was removed by filtration, and the filtrate was freeze-dried. Thus, Compound 24 (0.36 g) represented by the following Formula (13) was obtained. The tacrolimus content was calculated to be 21%. 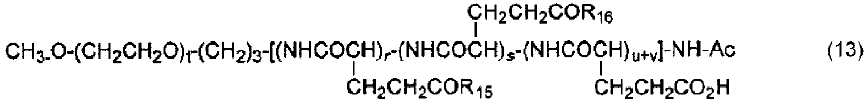 wherein **R**₁₅ represents a tacrolimus residue; **R**₁₆ represents an isopropylaminocarbonylisopropylamino group; the average value of t is 272; the average value of r+s+u+v is 22; and the average value of r is 5.5.

### Synthesis Example 8

### Polymeric derivative labeled with 2-(2-aminoethoxy)-9-(diethylamino)-5H-benzo[a]phenoxazin-5-one (Compound 24)

DMF (50 mL) was added to PEG-pGlu-Ac (PEG: average molecular weight 12,000; polyglutamic acid: average degree of polymerization 22) (2.03 g) produced by the method described in Japanese Patent No. 4745664, and the mixture was dissolved at 35°C. 2-(2-Aminoethoxy)-9-(diethylamino)-5H-benzo[a]phenoxazin-5-one (59 mg) and DMT-MM (53 mg) were added to the solution at 25°C, and the mixture was stirred overnight. After 22 hours, DMT-MM (26 mg) was added to the mixture, and the resulting mixture was stirred for 1.5 hours. The reaction liquid was added dropwise to a mixed solution of diisopropyl ether (480 mL) and ethanol (120 mL) for 15 minutes, and the mixture was stirred for 4 hours at room temperature. A precipitate was collected by filtration and washed with a mixed solution (100 mL) of diisopropyl ether and ethanol, and then a precipitate thus obtained was dried. Thus, a polymeric derivative (1.89 g) represented by the following Formula (14) was obtained. 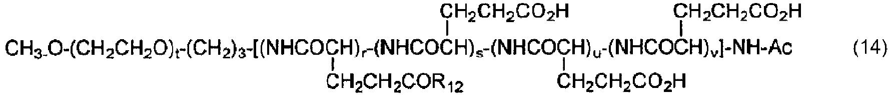 wherein the average value of t is 272; the average value of r+s+u+v is 22; the average value of r is 1; the average values of s and u are each 0; the average value of v is 21; and **R**₁₂ represents 2-(2-aminoethoxy)-9-(diethylamino)-5H-benzo[a]phenoxazin-5-one residue.

DMF (10 mL) was added to the polymeric derivative thus obtained (0.50 g), 4-phenylbutylamine (60 µL), and DMAP (84 mg), and the mixture was dissolved at 35°C. DIPCI (211 µL) was added to the solution at room temperature, and the mixture was stirred overnight. After 23.5 hours, DIPCI (106 µL) was added thereto, and the mixture was stirred for 2 hours. The reaction liquid was added dropwise to a mixed solution of diisopropyl ether (200 mL) and ethanol (50 mL), and the mixture was stirred for 4 hours at room temperature. A precipitate was collected by filtration and washed with a mixed solution (50 mL) of diisopropyl ether and ethanol, and then a precipitate thus obtained was dissolved in acetonitrile (15 mL). Subsequently, purified water (5 mL) and an ion exchange resin (DOWEX 50 (H⁺) manufactured by Dow Chemical Company, 10 mL) was added to the solution. After the mixture was stirred for 3 hours, the ion exchange resin was removed by filtration, and the filtrate was freeze-dried. Thus, Compound 24 (0.50 g) represented by the following Formula (15) was obtained. The content of 2-(2-aminoethoxy)-9-(diethylamino)-5H-benzo[a]phenoxazin-5-one of Compound 24 was calculated to be 2%. 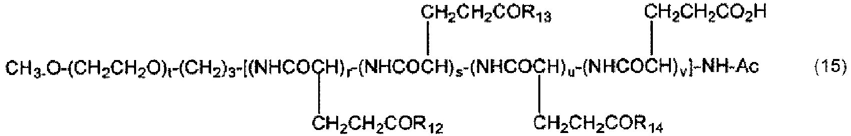 wherein the average value of t is 272; the average value of r+s+u+v is 22; the average value of r is 1; the average value of s is 10; **R**₁₂ represents a 2-(2-aminoethoxy)-9-(diethylamino)-5H-benzo[a]phenoxazin-5-one residue; **R**₁₃ represents a 4-phenylbutylamino group; and **R**₁₄ represents an isopropylaminocarbonylisopropylamino group.

The content of 2-(2-aminoethoxy)-9-(diethylamino)-5H-benzo[a]phenoxazin-5-one according to the present invention was calculated from the consumption rate of 2-(2-aminoethoxy)-9-(diethylamino)-5H-benzo[a]phenoxazin-5-one in the reaction solution measured under the following HPLC conditions.

### Analysis conditions for HPLC

Column: INERTSIL ODS-3, 4.6φ × 150 mm
column temperature: 40°C
eluent liquid A: 0.1% aqueous solution of phosphoric acid, liquid B: acetonitrile
gradient: liquid B (%) (time, minutes), 10(0), 10(0.01), 50(12.0), 50(15.0), 10(15.01), stop (20.0)
flow rate: 1.0 mL/min
detector (detection wavelength): UV (254 nm)

The tacrolimus content of the compound according to the present invention was calculated from the consumption rate of tacrolimus in the reaction solution measured under the following HPLC conditions.

### Analysis conditions for HPLC

Column: SHIM-PACK XR-ODSIII, 2.0φ × 200 mm
Column temperature: 40°C
Eluent liquid A: 0.1% aqueous solution of phosphoric acid, liquid B: acetonitrile, liquid A/liquid B = 80/20
Flow rate: 0.5 mL/min
Detector (detection wavelength): UV (254 nm)

### Test Example 1

### Test on drug releasability into phosphate buffered physiological saline

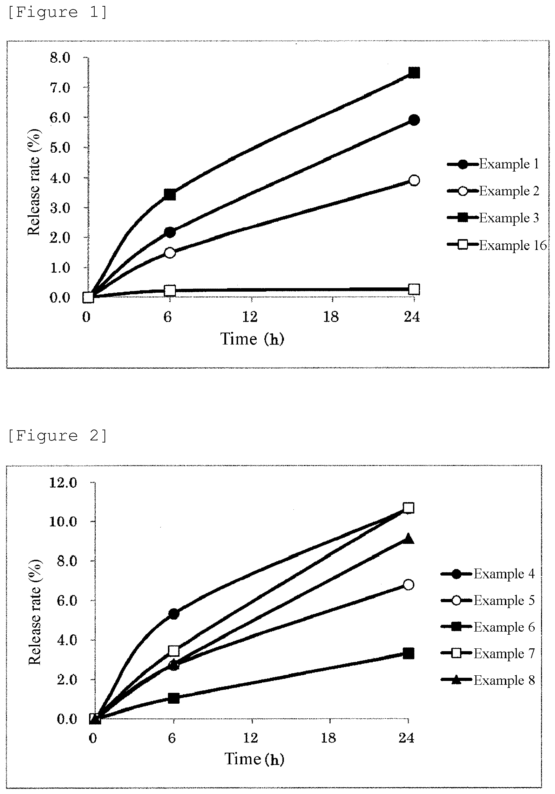

The compounds of Examples 1 to 8 and Example 16 were respectively dissolved in phosphate buffered physiological saline (pH 7.4) to a concentration of 1.0 mg/mL, and the solutions were left to stand at a constant temperature of 37°C. The amount of released tacrolimus was measured over time by HPLC, and the proportion of the amount of released tacrolimus with respect to the total amount of tacrolimus in the compound used was determined. The results for Examples 1 to 3 and Example 16 are presented in Fig. 1, and the results for Examples 4 to 8 are presented in Fig. 2.

As a result, it was found that the compounds of the present invention slowly released the drug in the absence of any enzyme.

### Test Example 2

### Blood concentration profile in rats

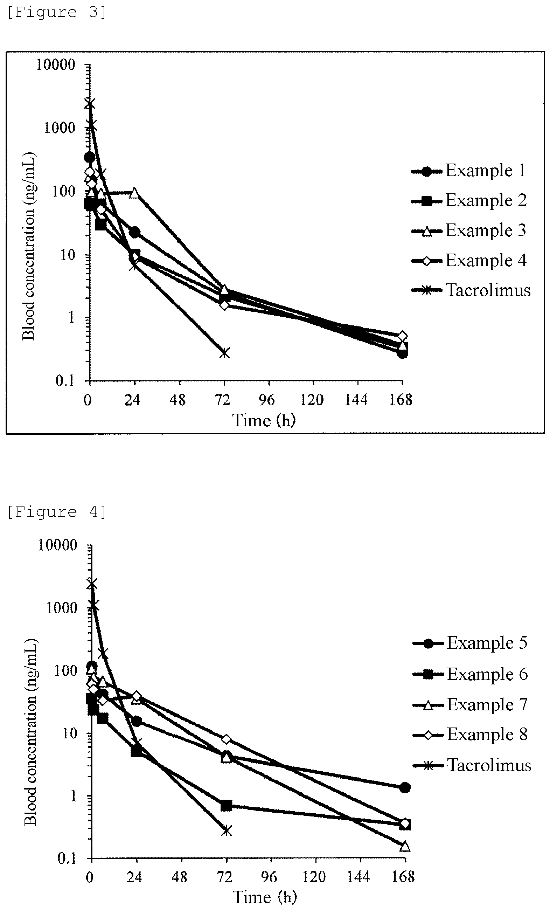

Eight-week old female SD rats (Charles River Laboratories Japan, Inc.) were grouped such that each group included two animals, and the rats were intravenously administered with tacrolimus or the compounds of Examples 1 to 8 once at a dose of 5 mg/kg through the caudal vein. After 5 minutes, 1 hour, 6 hours, 24 hours, 72 hours, and 168 hours after administration (for tacrolimus, up to 72 hours), the jugular vein was exposed under isoflurane anesthesia, blood was collected successively in an amount of 0.3 mL each time, and the tacrolimus concentration in the collected blood was measured. The results for Examples 1 to 4 and tacrolimus are presented in Fig. 3, and the results for Examples 5 to 8 and tacrolimus are presented in Fig. 4. Furthermore, the blood concentration parameters for the various compounds are presented in Table 3. However, the results for Examples 1 to 8 are the concentrations and parameters for tacrolimus cut out from micelles.

**[Table 3]**

| Group | Dose (mg/mL) | T1/2 (hr) | Tmax (hr) | Cmax (ng/mL) | AUCinf. (hr·ng/ml) | MRTinf. (hr) |
|---|---|---|---|---|---|---|
| Example 1 | 5 | 30.4 | 0.083 | 343 | 1980 | 20.5 |
| Example 2 | 5 | 35.5 | 0.083 | 63.6 | 956 | 30.2 |
| Example 3 | 5 | 44.7 | 0.083 | 171 | 3480 | 22.9 |
| Example 4 | 5 | 59.0 | 0.083 | 201 | 1360 | 26.5 |
| Example 5 | 5 | 56.3 | 0.083 | 117 | 1600 | 48.7 |
| Example 6 | 5 | 92.8 | 0.083 | 36.5 | 508 | 50.8 |
| Example 7 | 5 | 18.5 | 0.083 | 107 | 2170 | 24.8 |
| Example 8 | 5 | 21.2 | 0.083 | 62.3 | 2110 | 35.7 |
| Tacrolimus | 5 | 7.51 | 0.083 | 2430 | 5400 | 4.12 |

From these results, Examples 1 to 8 show prolongation of the blood concentration half-life and the MRTinf., compared to tacrolimus, and it is obvious that retentivity in blood was enhanced compared to tacrolimus alone.

### Test Example 3

### Test on single-dose toxicity in rats

Female DA rats (Japan SLC, Inc.) were intravenously administered through the caudal vein with tacrolimus or Example 1, each once in the dosage indicated in Table 4 and Table 5, and an observation of general symptoms was made successively. The relative bodyweights at the time of administration of Example 1 are presented in Fig. 5, and the relative bodyweights at the time of administration of tacrolimus are presented in Fig. 6.

**[Table 4]**

| Test substance | Dosage (mg/kg) | Volume (mL/kg) | Concentration (mg/mL) | Number of animals | Age in weeks |
|---|---|---|---|---|---|
| Solvent control (Cremophor-ethanol mixed liquid) | - | 10 | - | 1 | 13 |
| Tacrolimus (iv) | 20 | 10 | 2 | 2 | 13 |
| Tacrolimus (iv) | 30 | 10 | 3 | 2 | 13 |

**[Table 5]**

| Test substance | Dosage (mg/kg) | Volume (mL/kg) | Concentration (mg/mL) | Number of animals | Age in weeks |
|---|---|---|---|---|---|
| Solvent control (physiological saline) | - | 10 | - | 2 | 9 |
| Example 1 | 20* | 10 | 2* | 2 | 9 |
| Example 1 | 30* | 10 | 3* | 2 | 9 |
| Example 1 | 40* | 10 | 4* | 2 | 9 |
| Example 1 | 50* | 10 | 5* | 2 | 9 |

| | | | | | |
|---|---|---|---|---|---|
| *: in terms of tacrolimus | | | | | |

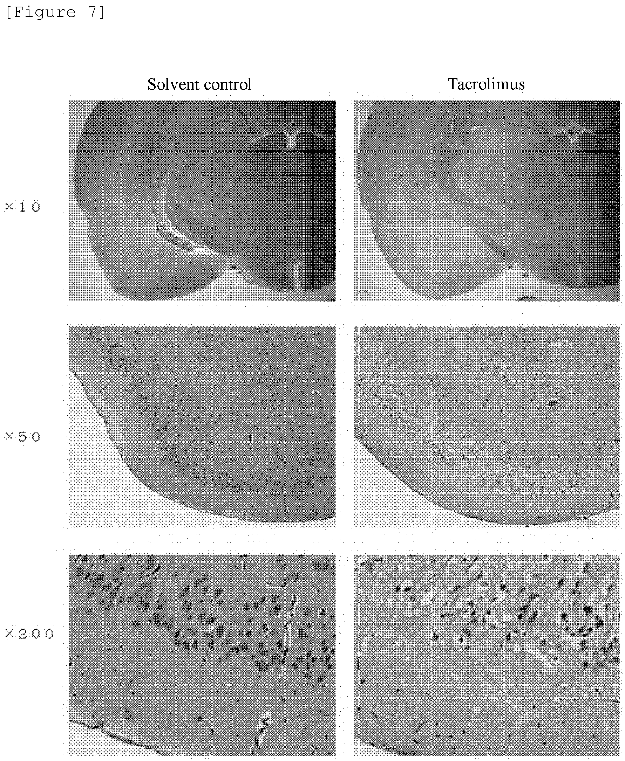

In the tacrolimus-administered group, a prone position, sedation, and a decrease in locomotor activity were recognized immediately after administration at a dose of 20 mg/kg or more. On the second day after administration, a decrease in locomotor activity, a prone position, salivation, convulsion, staggering gait, and irregular respirations were recognized, and half the animals in each group died. In the cerebral cortex of the dead rats, neuronal vacuolation was recognized, as shown in Fig. 7. Meanwhile, in the rats administered with the compound of Example 1, no death cases were observed even in the 50 mg/kg-administered group, which was the highest dose.

From the results of Test Example 3, it can be said that the lethal dose of tacrolimus is lower than 20 mg/kg, and the lethal dose of the compound of Example 1 is higher than 50 mg/kg. The tacrolimus-administered group showed a possibility of death caused by central nervous system disorders. Meanwhile, it was speculated that since the compound of Example 1 had a lower Cmax than tacrolimus as a result of polymeric derivatization of tacrolimus, migration property of the drug to the brain was suppressed, and the compound of Example 1 could be administered in a higher dosage than in the case of tacrolimus.

### Test Example 4

### Accumulation of fluorescence-labeled polymeric derivative at inflammation site in DSS-induced colitis mice

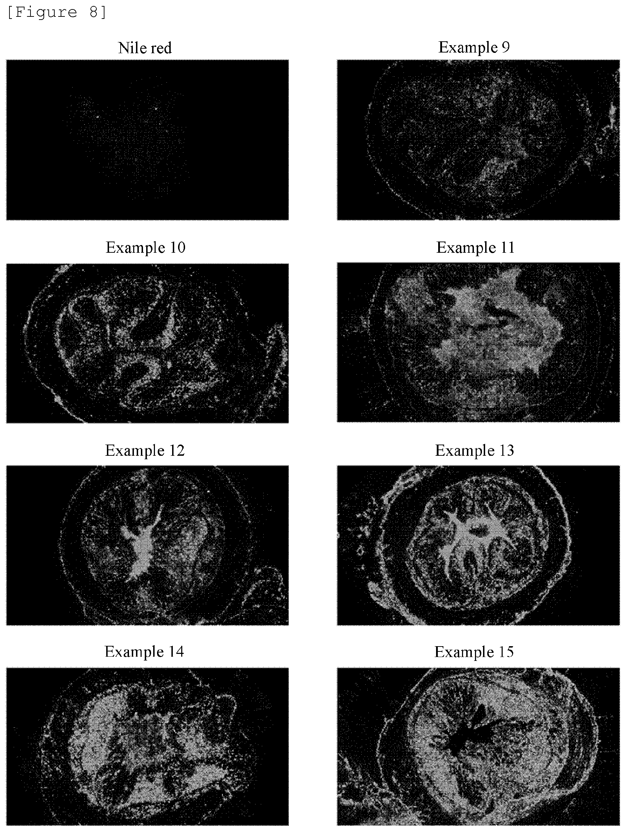

C57BL/6J mice (Charles River Laboratories Japan, Inc.) were allowed to freely drink a 2% dextran sulfate sodium (DSS) solution with water, and thereby ulcerative colitis was induced in the animals. On the fourth day after the mice were allowed to drink the 2% DSS solution, the mice were intravenously administered through the caudal vein with a physiological saline solution of each of the compounds of Examples 9 to 15 (5 mg/kg), the administration being performed in three mice in each group. As a control drug, Nile Red was dissolved in anhydrous ethanol and Cremophor, the solution was diluted with physiological saline, and the dilution was intravenously administered through the caudal vein. After 24 hours from the administration, frozen pathological sections of the colon were produced, and an observation of fluorescence was made. The results are presented in Fig. 8.

From the results of Test Example 4, it is obvious that Examples 9 to 15 accumulated to a large extent at the inflammation sites of the colon, compared to the control drug, Nile Red.

### Test Example 5

### Anti-inflammatory effect for rat collagen-induced arthritis (1)

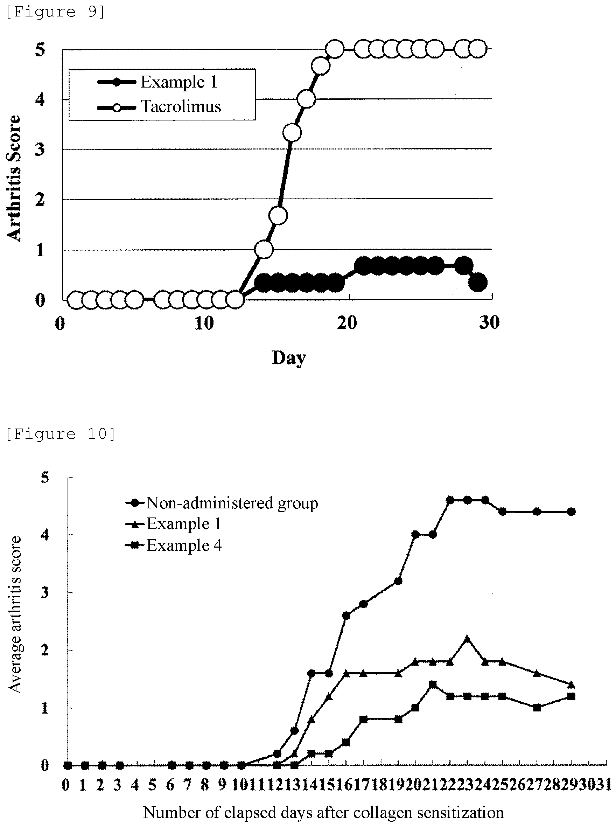

0.3 mg of bovine articular cartilage-derived type II collagen (immunological grade: Collagen Research Center, Ltd.) was intradermally administered on the dorsal side of nine-week old female DA rats (Japan SLC, Inc.), and thereby collagen-induced arthritis was induced in the rats. On the day of type II collagen sensitization and on the 7^{th} day, 14^{th} day, and 21^{st} day after sensitization, a physiological saline solution of the compound of Example 1 (5 mg/kg) was intravenously administered to three animals in each group through the caudal vein. As a control drug, tacrolimus hydrate was dissolved in anhydrous ethanol and Cremophor, and then the solution was diluted with physiological saline. The dilution was intravenously administered through the caudal vein. Determination of arthritis was made by scoring based on visual inspection. The results are presented in Fig. 9.

### Test Example 6

### Anti-inflammatory effect on rat collagen-induced arthritis (2)

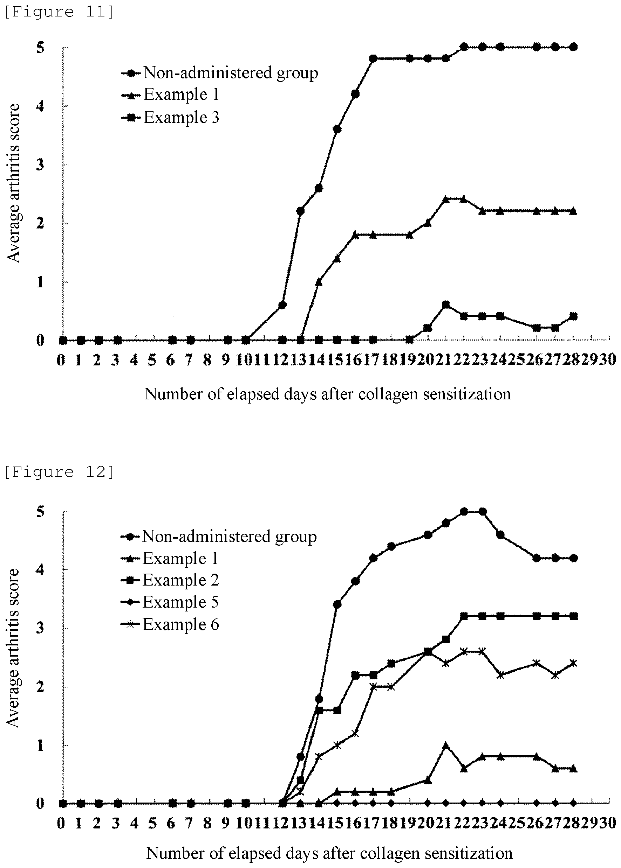

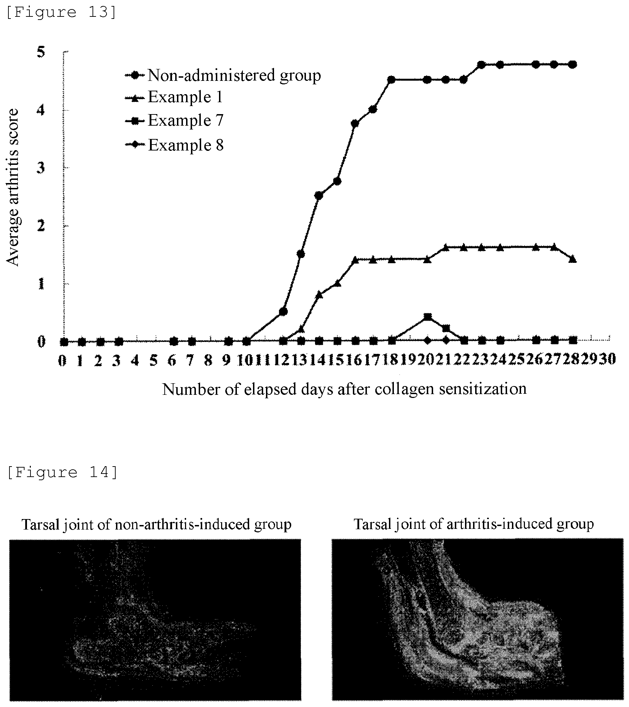

A physiological saline solution of each of the compounds of Examples 1 to 8 (5 mg/kg) was intravenously administered to five animals in each group through the caudal vein. As a control, a non-administered group was set up. Except for those, the experiment was carried out in the same manner as in Test Example 5, and the anti-inflammatory effect was investigated. The results of the compounds of Examples 1 and 4 are presented in Fig. 10; the results for the compounds of Examples 1 and 3 are presented in Fig. 11; the results for the compounds of Examples 1, 2, 5, and 6 are presented in Fig. 12; and the results for the compounds of Examples 1, 2, 7, and 8 are presented in Fig. 13.

From the above-described results, it was confirmed that the compounds of the present invention slowly released the drug over a long time period and thus enhanced the blood retentivity of tacrolimus. Furthermore, the compounds of the present invention exhibited high accumulability at inflammation sites and had a superior arthritis suppressing effect at a lower dosage and for a longer interval of administration. Furthermore, it was found that the compounds of the present invention reduce toxicity by maintaining the maximum blood concentration at a low dose.

### Test Example 7

### Accumulation of fluorescence-labeled polymeric derivative at inflammation site in rat collagen-induced arthritis

0.3 mg of bovine articular cartilage-derived type II collagen was intradermally administered to ten-week old female DA rats (Japan SLC, Inc.), and thereby collagen-induced arthritis was induced in the rats. For the collagen-induced arthritis-induced group and non-induced group, the compound of Synthesis Example 8 was intravenously administered through the caudal vein, and after 24 hours from the administration, frozen pathological sections of hind limb tarsal joint were produced, and an observation of fluorescence was made. The results are presented in Fig. 14.

As a result, it became clear that the compound of Synthesis Example 8 accumulated at a higher concentration at the tarsal joint of the arthritis-induced group, compared to the non-arthritis-induced group. Furthermore, in the arthritis-induced group, it was confirmed that the compound of Synthesis Example 8 accumulated at sites where acute inflammation was recognized, such as peripheral edematous sites and inflammatory cell infiltration sites.

## Claims

1. A polymeric derivative of tacrolimus, comprising a copolymer of a polyethylene glycol segment and a polyamino acid derivative, wherein a carboxy group in a side chain of the copolymer is bonded to an alcoholic hydroxy group of tacrolimus.

2. The polymeric derivative of tacrolimus according to claim 1, wherein the polyamino acid derivative is an aspartic acid derivative.

3. The polymeric derivative of tacrolimus according to claim 1 or 2, wherein the polymeric derivative of tacrolimus is represented by the following General Formula (1): 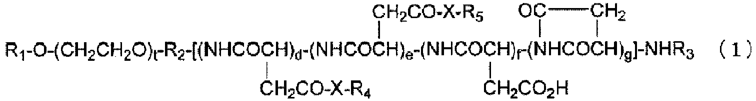 wherein **R**₁ represents a hydrogen atom or a (C1-C6) alkyl group; **R**₂ represents a linking group; **R**₃ represents a hydrogen atom or a (C1-C6) acyl group; **R**₄ represents a residue of an alcoholic hydroxy group of tacrolimus; **R**₅'s are each independently selected from the group consisting of a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group; **X** represents a bond or a linking group; t represents an integer from 5 to 11,500; d represents an integer from 1 to 200; e, f, and g each represent zero or a positive integer of 200 or less; d+e+f+g represents an integer from 3 to 200; and the order of arrangement of the various repeating units of the polyamino acid derivative is arbitrary.

4. The polymeric derivative of tacrolimus according to claim 1 or 2, wherein the polymeric derivative of tacrolimus is represented by the following General Formula (2): 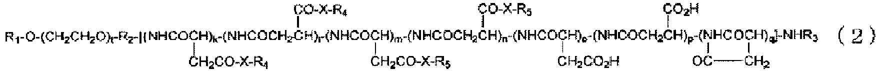 wherein **R**₁ represents a hydrogen atom or a (C1-C6) alkyl group; **R**₂ represents a linking group; **R**₃ represents a hydrogen atom or a (C1-C6) acyl group; **R**₄ represents a residue of an alcoholic hydroxy group of tacrolimus; **R**₅'s are each independently selected from the group consisting of a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group; **X** represents a bond or a linking group; t represents an integer from 5 to 11,500; k, 1, m, n, o, p, and q each represent zero or a positive integer of 200 or less; k+l represents an integer from 1 to 200; k+l+m+n+o+p+q represents an integer from 3 to 200; and the order of arrangement of the various repeating units of the polyamino acid derivative is arbitrary.

5. The polymeric derivative of tacrolimus according to claim 3 or 4, wherein **X** represents a bond.

6. The polymeric derivative of tacrolimus according to claim 5, wherein **R**₅ represents a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group.

7. The polymeric derivative of tacrolimus according to claim 3 or 4, wherein the linking group of **X** is an aspartic acid derivative.

8. The polymeric derivative of tacrolimus according to claim 7, wherein **R**₅ represents a hydrophobic substituent and -N(**R**₆)CONH(**R**₇), wherein **R**₆ and **R**₇, which may be identical or different, each represent a (C3-C6) cyclic alkyl group or a (C1-C5) alkyl group which may be substituted with a tertiary amino group.

9. The polymeric compound of tacrolimus according to claim 3 or 4, wherein the linking group of **X** is represented by the following General Formula (3) or General Formula (4): 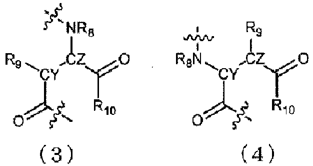 wherein **R**₈ and **R**₉ each independently represent a hydrogen atom or a (C1-C8) alkyl group; **R**₁₀ represents one or more groups selected from the group consisting of NH₂, a linear, branched or cyclic (C1-C20) alkylamino group which may have a substituent, a linear, branched or cyclic (C7-C20) aralkylamino group which may have a substituent, a (C5-C20) aromatic amino group which may have a substituent, and an amino acid residue having a protected carboxy group; and C**Y-**C**Z** represents CH-CH or C=C (double bond).

10. The polymeric compound of tacrolimus according to claim 9, wherein **R**₈ and **R**₉ are both a hydrogen atom, and C**Y**-C**Z** is CH-CH.

11. The polymeric derivative of tacrolimus according to any one of claims 3 to 10, wherein the hydrophobic substituent is selected from the group consisting of a (C1-C30) alkoxy group, a (C1-C30) alkenyloxy group, a (C1-C30) alkylamino group, a (C2-C60) dialkylamino group, a (C1-C30) alkenylamino group, and an amino acid derivative residue.

12. The polymeric derivative of tacrolimus according to any one of claims 3 and 5 to 11, wherein **R**₁ represents a (C1-C6) alkyl group; **R**₂ represents a (C2-C6) alkylene group; **R**₃ represents a (C1-C6) acyl group; t represents an integer from 50 to 1,500; and d+e+f+g represents an integer from 4 to 150.

13. The polymeric derivative of tacrolimus according to any one of claims 3 and 5 to 11, wherein **R**₁ represents a (C1-C3) alkyl group; **R**₂ represents (C2-C4) alkylene group; **R**₃ represents a (C1-C3) acyl group; t represents an integer from 100 to 1,500; and d+e+f+g represents an integer from 8 to 120.

14. The polymeric derivative of tacrolimus according to any one of claims 4 to 11, wherein **R**₁ represents a (C1-C6) alkyl group; **R**₂ represents a (C2-C6) alkylene group; **R**₃ represents a (C1-C6) acyl group; t represents an integer from 50 to 1,500; and k+l+m+n+o+p+q represents an integer from 4 to 150.

15. The polymeric derivative of tacrolimus according to any one of claims 4 to 11, wherein **R**₁ represents a (C1-C3) alkyl group; **R**₂ represents a (C2-C4) alkylene group; **R**₃ represents a (C1-C3) acyl group; t represents an integer from 100 to 1,500; and k+l+m+n+o+p+q represents an integer from 8 to 120.

16. The polymeric derivative of tacrolimus according to any one of claims 3 to 15, wherein **R**₁ represents a methyl group; **R**₂ represents a trimethylene group; and **R**₃ represents an acetyl group.

17. A method for preparing the polymeric derivative of tacrolimus according to any one of claims 1 to 16, the method comprising forming an ester-bond between an alcoholic hydroxy group of tacrolimus and a carboxy group in a side chain of a copolymer of a polyethylene glycol segment and a polyaspartic acid derivative by using a dehydration condensing agent in an organic solvent.

18. A macrolide immunosuppressant comprising the polymeric derivative of tacrolimus according to any one of claims 1 to 17 as an active ingredient.
